# EUROPEAN PATENT SPECIFICATION

(11) **EP 3 843 705 B1**
(45) Date of publication and mention of the grant of the patent: **29.11.2023**
(21) Application number: 19756406.5
(22) Date of filing: 27.08.2019
(51) Int. Cl.: A61K 9/14, A61K 9/51, A61K 31/365, A61K 31/7048, A61P 35/00

(54) **PREPARATION OF NANOSUSPENSION COMPRISING NANOCRYSTALS OF ACTIVE PHARMACEUTICAL INGREDIENTS WITH LITTLE OR NO STABILIZING AGENTS**
HERSTELLUNG VON NANOSUSPENSIONSHALTIGEN NANOKRISTALLEN VON PHARMAZEUTISCHEN WIRKSTOFFEN MIT GERINGEN ODER KEINEN STABILISATOREN
PRÉPARATION DE NANOSUSPENSION COMPRENANT DES NANOCRISTAUX D'INGRÉDIENTS PHARMACEUTIQUES ACTIFS COMPORTANT PEU OU PAS D'AGENTS DE STABILISATION

(30) Priority: 27.08.2018 EP 18306138
(43) Date of publication of application: 07.07.2021
(73) Proprietor: Centre National de la Recherche Scientifique (CNRS), 75016 Paris (FR); Université Paris Cité, 75006 Paris (FR); INSTITUT NATIONAL DE LA SANTE ET DE LA RECHERCHE MEDICALE - INSERM, 75013 Paris (FR); Ecole Nationale Superieure de Chimie Paris, 75231 Paris Cedex 05 (FR)
(72) Inventor: MARTIN, Brice, 94160 SAINT MANDE (FR); MIGNET, Nathalie, 92140 CLAMART (FR); CORVIS, Yohann, 92150 SURESNES (FR)
(74) Representative: A.P.I. Conseil
(86) International application number: PCT/EP2019/072873
(87) International publication number: WO 2020/043735

(56) References cited:
- CN-A- 107 303 272
- SONG JU ET AL: "Development and Characterisation of Ursolic Acid Nanocrystals Without Stabiliser Having Improved Dissolution Rate and In Vitro Anticancer Activity", AAPS PHARMSCITECH, vol. 15, no. 1, 11 September 2013 (2013-09-11), pages 11-19, XP055554118, DOI: 10.1208/s12249-013-0028-0
- DATABASE EMBASE [Online] ELSEVIER SCIENCE PUBLISHERS, AMSTERDAM, NL; October 2015 (2015-10), GE Z -Q ET AL: "Enhanced oral bioavailability of ursolic acid nanoparticles via antisolvent precipitation with TPGS1000 as a stabilizer", XP002788864, Database accession no. EMB-2015405373 & GE Z -Q ET AL: "Enhanced oral bioavailability of ursolic acid nanoparticles via antisolvent precipitation with TPGS1000 as a stabilizer", JOURNAL OF DRUG DELIVERY SCIENCE AND TECHNOLOGY 20151001 EDITIONS DE SANTE FRA, vol. 29, 1 October 2015 (2015-10-01), pages 210-217, ISSN: 1773-2247
- KHAN SHAHZEB ET AL: "Nanocrystal Preparation: Low-Energy Precipitation Method Revisited", CRYSTAL GROWTH & DESIGN., vol. 13, no. 7, 5 June 2013 (2013-06-05), pages 2766-2777, XP055554827, US ISSN: 1528-7483, DOI: 10.1021/cg4000473
- BAJAJ AMRITA ET AL: "Nanocrystallization by Evaporative Antisolvent Technique for Solubility and Bioavailability Enhancement of Telmisartan", AAPS PHARMSCITECH, SPRINGER NEW YORK LLC, US, vol. 13, no. 4, 28 September 2012 (2012-09-28), pages 1331-1340, XP035148014, ISSN: 1530-9932, DOI: 10.1208/S12249-012-9860-X
- POURETEDAL HAMID REZA: "Preparation and characterization of azithromycin nanodrug using solvent/antisolvent method", INTERNATIONAL NANO LETTERS, vol. 4, no. 1, March 2014 (2014-03), pages 1-9, XP055554836, London, UK ISSN: 2008-9295, DOI: 10.1007/s40089-014-0103-x
- MANSOURI MANSOUR ET AL: "Preparation and Characterization of Ibuprofen Nanoparticles by using Solvent/ Antisolvent Precipitation", OPEN CONFERENCE PROCEEDINGS JOURNAL, vol. 2, no. 1, 7 July 2011 (2011-07-07), pages 88-94, XP055555255, NL ISSN: 2210-2892, DOI: 10.2174/2210289201102010088
- Yarraguntla Srinivasa Rao ET AL: "Formulation and Evaluation of Lornoxicam Nanocrystals with Different Stabilizers at Different Concentrations", Asian Journal of Pharmaceutics, July 2016 (2016-07), pages 198-207, XP055554913, Mandsaur DOI: 10.22377/ajp.v10i3.727 Retrieved from the Internet: URL:http://www.asiapharmaceutics.info/inde x.php/ajp/article/viewFile/727/466 [retrieved on 2019-02-11]

## Description

### Field of the invention

The invention relates to a method for manufacturing a nanostructured powder of an Active Pharmaceutical Ingredient (API), said nanostructured powder, a method for preparing a nanosuspension from said nanostructured powder and nanosuspension obtained thereof.

### Background of the invention

About 70% of drug molecules face problems of poor bioavailability and instability. The prominent reason behind these issues is poor aqueous solubility, and the resulting low bioavailability. Formulation of insoluble drugs using co-solvents is one of the oldest and widely used technique, especially for liquid formulation intended for oral and intravenous administration. However, the are many others insoluble drug delivery strategies, such as chemical modification of the drug, to obtain various forms of the drugs (ester/salt), prodrugs or active metabolites of drugs.

Decreasing particle size in drug powders is another approach to overcome this challenge. Widely used, the micronization of drug powders to sizes between 1 and 10 µm in order to increase the surface area, and thus the dissolution velocity, is often not sufficient to overcome bioavailability problems of many very poorly soluble drugs. A consequent step was to move from micronization to nanonization i.e. reducing drug particle size to sub-micron range. Over the past two decades, nanoparticle technology, e.g. the use of nanocrystals instead of microcrystals for oral bioavailability enhancement, but also the use of nanocrystals suspended in water (nanosuspensions) for intravenous or pulmonary drug delivery, has become a wellestablished and proven formulation approach for poorly-soluble drugs. In the field of pharmaceuticals, the term 'nanoparticle' is applied to structures less than 1 µm in size for at least one of their dimensions. Drug nanoparticles can be produced by various technologies, which can be broadly categorized into 'bottom up' and 'top-down' technologies.

Wet milling is a top-down approach in the production of small drug particles in which a mechanical energy is applied to physically break down coarse particles to smaller ones using beads. The pearls or balls used to mill consist of ceramic (cerium or yttrium-stabilized zirconium oxide), stainless steel, glass, or highly cross-linked polystyrene resin-coated beads. A major drawback of the wet milling technique is the erosion of the balls arising from the intensive mixing forces in the vessel. Residues of the milling media produced from erosion may result in product contamination, leading to chemical destabilization of the newly-formed particle surfaces and possibly affecting critical product attributes such as particle size and size distribution. Another problem associated with wet milling is the loss of drug during the milling by the action of temperature and mechanical action. Thus, the milling process does not represent the ideal way for the production of small particles because drug substance properties and surface properties are altered in a mainly uncontrolled manner. High pressure homogenization is another top-down approach, in which size reduction of drug particles is achieved by repeatedly cycling, to 200 plus cycles, with the aid of a piston, a drug suspension through a very thin gap at high velocity, around 500 m/s, and under pressure, 1000-1500 bars. The extent of subdivision of the nanoparticles depends on the pressure applied as well as the number of homogenization cycles the drug suspension is subjected to during the process. A drawback of high pressure homogenization is its energy intensity which may result to high temperature process leading to possible degradation of the components. On the other hand, liquid antisolvent is a bottom-up technique based on the addition of a drug solubilized in solvent to an antisolvent in which the drug is poorly or not soluble, thereby precipitating the drug in the form of nanoparticles. However, this technique allows a poor control over particle size distribution, this parameter being generally modulated throughout the addition of stabilizers such as surfactants, polymers or electrolytes in the solvent and/or antisolvent.

Among active pharmaceutical ingredients, etoposide is a cancer drug poorly soluble in water. Although it contains a carbohydrate portion, etoposide is substituted with lipophilic groups which negate much of the hydrophilic character that carbohydrates normally impart to a drug molecule. Examples of commercially available oral and injectable etoposide formulations are Vepesid^{®} and Toposar^{®}. Vepesid^{®} formulation comprises etoposide solubilized in a cosolvent mixture of PEG 400, glycerin, citric acid, and water (Strickley, 2004. Pharmaceutical Research, Vol. 21, No. 2 page 201-230). TOPOSAR^{®} formulation comprises etoposide as a 20 mg/mL, 2 mg/ml citric acid, 30 mg/ml benzyl alcohol, 80 mg/ml polysorbate 80/tween 80, 650 mg/ml polyethylene glycol 300, and 30.5% (v/v) alcohol. To limit toxicity, both formulations must be diluted prior to use and slowly infused.

Wet milling of etoposide makes it possible to obtain etoposide nanosuspension. In particular, Merisko et al. (Pharmaceutical Research, Vol. 13, No. 2, 1996) describes the preparation of an etoposide nanocrystalline suspension wherein an etoposide powder is wet milled using a zirconia 2%w/v solid suspension containing 1%w/v Pluronic^{®} F-127 as surfactant stabilizer. After four days of milling, etoposide nanosuspensions were harvested. However, this process is long and energy intensive. Moreover, the process inherently results in contamination due to milling material and degradation of etoposide biological activity due to thermal energy released during the milling.

### Summary of the invention

The aim of the present invention is to provide a nanostructured powder containing active pharmaceutical ingredient (API) agglomerates containing API in its crystalline form. The nanostructured powder of the invention has a low level/no stabilizing agent and is free of contaminants typically produced during wet milling techniques. The nanostructure powder produced is therefore particularly well suited for medical use. The nanostructured powder is stable and can be stored before being used and before being dispersed in nanosuspension.

Thus, a first object of the invention is to supply a method for manufacturing a nanostructured powder comprising nanocrystalline agglomerates containing active pharmaceutical ingredient (API) in its crystalline form, said method comprising:
(i) Preparing a first solution comprising API and an API solvent;
(ii) Mixing the first solution with a second solution comprising an API antisolvent and optionally a stabilizing agent P1 to obtain a third mixture which is a miscible liquid mixture containing the API solvent, the API antisolvent, and solubilized API;
(iii) Evaporating said miscible liquid mixture until both the API solvent and the API antisolvent are evaporated, advantageously under vacuum;
characterized in that
- when the stabilizing agent P1 is present, the miscible liquid mixture has a stabilizing agent P1 to API weight ratio equal or less than 5, preferably less than 2,
- the API antisolvent volume to API solvent volume ratio is at least of 4.5, preferably at least of 5,
- the method does not comprise any intermediate step between steps (ii) and (iii), in particular any filtration or any other step intended to eliminate micrometric particles, and
- the API nanocrystals form and/or develop during step (iii).

A second object of the invention is to provide a nanostructured powder comprising nanocrystalline agglomerates containing API in its crystalline form said agglomerates having at least one dimension of less than 1 µm.

Advantageously, said nanostructured powder further comprises a stabilizing agent in a stabilizing agent to API weight ratio equal or less than 5, preferably less than 2.

More advantageously, said nanostructured powder is free of stabilizing agent.

A third object of the invention is to supply a method for manufacturing a nanosuspension comprising API nanocrystals comprising a step of mixing a nanostructured powder as defined above, an API antisolvent and optionally a stabilizing agent P2 in a stabilizing agent P2 to API weight ratio equal or less than 5, preferably less than 2.

A fourth object of the invention is to provide a nanosuspension obtainable by the method above comprising API nanocrystals and an API antisolvent, wherein the API nanocrystals have an average size of less than 500 nm, preferably less than 200 nm, more preferably less than 100 nm.

Advantageously, the weight ratio of stabilizing agent to API is equal or inferior to 5, preferentially comprised between 0 and 2.

A fifth object of the invention is a nanosuspension of the invention for its use as a medicament, notably in the treatment of cancer.

Advantageously, said API is selected among podophyllotoxin and podophyllotoxin derivatives such as etoposide and teniposide.

### Legends to the figures

Figure 1: Evolution of etoposide nanocrystal size as a function of the weight ratio of poloxamer 407 (commercial name Pluronic F-127) as stabilizing agent P1 to etoposide as API.
Figure 2: Graph showing etoposide nanocrystals size vs. methanol to precipitation water volume ratio. The etoposide has been solubilized in methanol and then injected into different volumes of water without stabilizing agent P1 followed by a complete evaporation, to explore the impact of methanol to precipitation water volume ratio on the nanocrystal size.
Figures 3: Graphs showing raw correlation data measured by dynamic light scattering of etoposide NCs dispersed in water after 5 hours versus different amount of Pluronic F-127 (A, B) or Pluronic F-68 (C, D) as stabilizing agent P2, for stabilization in solution. The two curves correspond to two measurements of the sample.
Figure 4: SEM pictures of a marketed etoposide powder (A, B) and an etoposide nanostructured powder (C, D) of the invention.
Figure 5: Powder X-ray diffraction (PRDX) pattern performed on a marketed etoposide powder and an etoposide nanostructured powder of the invention. PRDX examinations were made to evidence and compare the crystallinity of etoposide nanocrystals powder after the precipitation process with the pure drug.
Figure 6: DSC curves obtained for a 10 °C/min scan rate A. the etoposide marketed powder, B. the Pluronic F-127 powder, C. an etoposide nanostructured powder of the invention form with 0.03% weight volume Pluronic F-127.
Figure 7: DSC curves obtained for a 5 °C/min scan rate.
Figure 8: Comparison of the DSC curves obtained for the etoposide NCs/P407 solid dispersion at 5 and 10 °C/min scan rates.
Figures 9A and 9B: Pictures of etoposide NCs/ Pluronic F-127 solid dispersion as a function of the temperature for two different sample mass (msample1 < msample2). Thermal microscopy examination was used to confirm the solid state of etoposide NCs in the Pluronic F-127 polymeric matrix.
Figure 10: Dissolution profiles of etoposide nanocrystals, microcrystals with P407 and Toposar.
Figure 11: Graphs showing the percentage of CT26 colon cancer cells viability after 48 and 72 hours incubation with etoposide nanocrystals of the invention with and without albumin. "Microcrystals" correspond to marketed etoposide powder directly sonicated in water.
Figure 12: TEM images for control CT26 cells (A), Etoposide NCs with 0,083% wt/v F-127 CT26 (B), Etoposide NCs with 0,083 wt/v F-127 + Albumin 0,2% wt/v CT26 (C). The nanocrystals are marked with an arrow.
Figure 13: TEM images of Podophyllotoxin NCs after redispersion in water with F-127 0.033% wt/v, 500 nm scale (A) and 0.5 µm scale (B).

### Detailed description of the invention

In the present invention, the term "stabilizing agent" refers to polymers or surfactants that are able to facilitate the formation of particles and/or stabilize the size of said particles. Without willing bound to a theory, it appears that these stabilizing agents adsorb to the surfaces of the particles, and (a) convert lipophilic to hydrophilic surfaces with increased steric hindrance/stability, and (b) possibly modify zeta potential of surfaces with more charge repulsion stabilization.

Such stabilizing agent may be selected from the group consisting of phospholipids (like phosphatidyl choline such as egg phosphatidylcholine, soy phosphatidylcholine in particular hydrogenated soy-lecithin such as those commercialized under trade name Phospholipon^{®}; synthetic derivatives of phophatidylcholine); lipid derivatives (like distearoyl-sn-glycero-3-phosphoethanolamine-N-[amino(polyethylene glycol)-1000, distearoyl-sn-glycero-3-phosphoethanolamine-N-[amino(polyethylene glycol)-2000, distearoyl-sn-glycero-3-phosphoethanolamine-N-[amino(polyethylene glycol)-5000); polysorbates; polymers, such as homopolymers, block and graft copolymers (like hydroxypropyl cellulose (HPC), hydroxypropyl methylcellulose (HPMC) and polyvinylpyrrolidone (PVP) such as PVP K-15, K-25, K-30, K-60 or K-90); nonionic tri-block copolymers, such as poloxamers; alkyl aryl polyether alcohol polymers (e.g. tyloxapol); gelatin; gum acacia; cholesterol; tragacanth; polyoxyethylene alkyl ethers; polyoxyethylene castor oil derivatives; polyoxyethylene sorbitan fatty acid esters; sorbitan fatty acid esters such as sorbitan trioleate 85; polyethylene glycols; polyoxyethylene stearates; mono and diglycerides; colloidal silicon dioxide; sodium dodecylsulfate; sodium lauryl sulfate magnesium aluminum silicate; triethanolamine; stearic acid; calcium stearate; glycerol monostearate; cetostearyl alcohol; cetomacrogol emulsifying wax; short and medium chain alcohols; polyols such as mannitol, propane-1,2,3-triol; polyvinyl alcohol and dioctyl sodium sulfosuccinate (DOSS) ; tocopheryl polyethylene glycol 1000 succinate (TGPS); sodium cholate, cholic acid, leucine vitamin E, chitosan, maltose, Asialofetuin, transferrin, albumin, cyclodextrin, fetal calf serum. Preferred examples of polysorbates are polysorbate 80 and polysorbate 20. It is further preferred that the stabilizing agent P2 is selected from the group consisting of polysorbate 80, polyvinylpyrrolidone K-30, hydroxypropylmethylcellulose, sodium lauryl sulfate, mannitol, lecithin, tocopheryl polyethylene glycol 1000, sorbitan trioleate 85, tyloxapol, poloxamer 338, phospholipon, sodium cholate, cholic acid, leucine, polyvinyl pyrrolidone- K25, distearoyl-sn-glycero-3-phosphoethanolamine-N-[amino(polyethylene glycol)-1000, distearoyl-sn-glycero-3-phosphoethanolamine-N-[amino(polyethylene glycol)-2000, distearoyl-sn-glycero-3-phosphoethanolamine-N-[amino(polyethylene glycol)-5000, vitamin E, tragacanth, decyl glucoside, fetal calf serum, soy phosphatidylcholine, egg phosphatidylcholine, a synthetic derivative of phophatidylcholine, dioctyl sulfosuccinate, chitosan, maltose, Asialofetuin, transferrin, and cyclodextrine, poloxamere 188, albumin, poloxamer 407 and mixtures thereof. Advantageously, the stabilizing agent is a poloxamer. The term "poloxamer" refers to a tri-block copolymer comprising or consisting of a central polyoxypropylene chain (also called polypropylene glycol, PPO) grafted on either side by a chain of polyoxyethylene (also known aspolyethylene glycol, POE). Poloxamers thus comprise a central hydrophobic chain of poly(propylene oxide) surrounded by two hydrophilic chains of poly (ethylene oxide) (PEO-PPO-PEO block copolymer). Poloxamers are generally designated by the letter "P" (for poloxamer) followed by three digits: the first two numbers multiplied by 100 gives the molecular weight of polyoxypropylene heart, and the last digit multiplied by 10 gives the percentage of content polyoxethylene. For example, P407 (also known as Pluronic^{®}F-127) is a poloxamer including the heart in a polyoxypropylene molecular mass of 4000 g / mol and a polyoxyethylene content of 70%. Preferably, poloxamer the stabilizing agent is a poloxamer having a hydrophilic lipophilic balance (HLB) ranging from about 18 to about 23. More advantageously, the stabilizing agent comprises a poloxamer or a mixture thereof selected from poloxamer 407 (or P407, one commercial trade name being commercial Pluronic F-127) or poloxamer 188 (or P188, one commercial trade name being Pluronic F-68) or poloxamer 338, or a mixture thereof. Even more advantageously, the stabilizing agent comprises a poloxamer having a hydrophilic lipophilic balance (HLB) ranging from about 18 to about 23 such as poloxamer 407.

More advantageously, the stabilizing agent is selected from polysorbate 80, polyvinylpyrrolidone, hydroxypropylmethylcellulose, sodium lauryl sulfate, mannitol, lecithin, tocopheryl polyethylene glycol 1000 succinate, sorbitan trioleate 85, tyloxapol, poloxamer 338, sodium cholate, cholic acid, leucine, distearoyl-sn-glycero-3-phosphoethanolamine-N-[amino(polyethylene glycol)-1000, distearoyl-sn-glycero-3-phosphoethanolamine-N-[amino(polyethylene glycol)-2000, distearoyl-sn-glycero-3-phosphoethanolamine-N-[amino(polyethylene glycol)-5000, vitamin E, tragacanth, decyl glucoside, fetal calf serum, soy phosphatidylcholine, egg phosphatidylcholine, a synthetic derivative of phophatidylcholine, dioctyl sulfosuccinate, chitosan, maltose, Asialofetuin, transferrin, albumin, and cyclodextrine, poloxamere 188, poloxamere 407, and mixtures thereof.

In the context of the invention, the term "stabilizing agent P1" (abbreviated "P1") refers to a stabilizing agent or a mixture thereof used in the method for manufacturing a nanostructured powder according to the invention. Without willing bound to a theory, it appears that during the precipitation step of liquid antisolvent precipitation processes, stabilizing agents are capable of retarding the growth and coalescence of API nanocrystals during the precipitation.

In the context of the invention, the term "stabilizing agent P2" (abbreviated "P2") refers to a stabilizing agent or a mixture thereof used in the method for manufacturing a nanosuspension according to the invention. Without willing bound to a theory, it appears that stabilizing agents are capable of adsorbing on surfaces of API nanocrystals and form a coating which retards API nanocrystals growth, and in certain case to stabilize morphology.

The stabilizing agent used in the method for manufacturing a nanostructured powder according to the invention (stabilizing agent P1) and the stabilizing agent used in the method for manufacturing a nanosuspension according to the invention (stabilizing agent P2) may be identical or different and are preferably selected from the stabilizing agents mentioned above.

### Nanostructured powder preparation

It is disclosed a method for manufacturing a nanostructured powder, comprising agglomerates of active pharmaceutical ingredient (API) nanocrystals, said method comprising said method comprising:
(i) Preparing a first solution comprising API and an API solvent;
(ii) Mixing the first solution with a second solution comprising an API antisolvent and optionally a stabilizing agent P1 to obtain a third mixture;
(iii) Evaporating the third mixture until both the API solvent and the API antisolvent are evaporated,
characterized in that when the stabilizing agent is present, the third mixture has a stabilizing agent P1 to API weight ratio equal or less than 5, preferably less than 2.

The method of the invention is particularly advantageous. It provides API yields of at least 80%, advantageously 90%. In the context of the invention, the API yield is defined as the mass ratio of API entering the process to the API coming out of the process, calculated as the mass of API into the first solution of step(i)): mass of API under the form of nanostructured powder at the end of step (iii).

### Step (i)

In the present invention, active pharmaceutical ingredients (API) are poorly soluble in water biologically useful compounds such as imaging agents, pharmaceutically useful compounds and in particular drugs for human and veterinary medicine. Poorly soluble compounds are those having typically a solubility in water is less than 5 mg/ml at a physiological pH of 6.5 to 7.4. Poorly soluble compounds may have a water solubility less than 1 mg/ml and even less than 0.1 mg/ml.

For example, API may be selected among podophyllotoxin, etoposide, teniposide, albendazole, amoitone B, amphotericin B, aprepitant, aripiprazole, ascorbyl palmitate, asulacrine, avanafil, azithromycin, baicalin, bexarotene, breviscapine, budenoside, buparvaquone, cabazitaxel, camptothecin, candesartan cilexetil, celecoxib, cilostazol, clofazimine, curcumin, cyclosporine, danazol, darunavir, dexamethasone, diclofenac acid, docetaxel, doxorubicin, efavirenz, fenofibrate, glibenclamide, griseofulvin, hesperetin, hydrocamptothecin, hydrocortisone, ibuprofen, indometacin, itraconazole, ketoprofen, loviride, lutein, mebendazole, mefenamic acid, meloxicam, methyltryptophan, miconazole, monosodium urate, naproxen, nimodipine, nisoldipine, omeprazole, oridonin, paclitaxel, piposulfan, prednisolone, puerarin, fisetin, resveratrol, riccardin D, rutin, silybin, simvastin, spironolactone, tarazepide and ziprasidone and mixtures thereof.

Advantageously, the API presents a log(P) comprised between 0 and 3. The octanol/water partition coefficient (P) is defined as the ratio of a chemical substance concentration in the octanol phase to its concentration in the aqueous phase of a two-phase octanol/water system (i.e. P = Concentration in octanol phase / Concentration in aqueous phase).

For example, API may be selected among teniposide, albendazole, amphotericin B, asulacrin, baicalin, breviscapine, budenoside, camptothecin, cilostazol, cyclosporine, darunazir, docetaxel, griseofulvin, hesperetin, hydrocamptothecin, hydrocortisone, medenbazole, monosodium urate, naproxen, omeprazole, oridonin, piposulfan, prednisolone, puerarin, fisetin, rutin, silybin, sprirolactone and mixture thereof.

Advantageously, the API is selected among podophyllotoxin and podophyllotoxin derivatives and mixtures thereof such as etoposide (CAS registry number : 33419-42-0) and teniposide (CAS registry number : 29767-20-2). Etoposide may be prepared for example as described in European patent specification No. 111058, or by processes analogous thereto. Teniposide may be prepared for example as described in PCT patent specification No. WO 93/02094, or by processes analogous thereto.

In the present invention, the term "API solvent" means a solvent or a mixture of solvents capable of dissolving the API and miscible with the API antisolvent used in the preparation.

Preferably, the API solvent is an organic solvent having a boiling point inferior to 300°C, preferably inferior to 200°C, more preferably inferior to 100°C, said boiling point being measured under atmospheric pression.

For example, the API solvent may be chosen among methanol, isopropanol, ethanol, acetonitrile, acetone, diethanolamine, diethylenetriamine, dimethylformamide, ethylamine, ethylene glycol, formic acid, furfuryl alcohol, glycerol, methyl diethanolamine, methyl isocyanide, N-Methyl-2-pyrrolidone, propanol, propylene glycol, pyridine, tetrahydrofuran, triethylene glycol and dimethyl sulfoxide and mixtures thereof.

Preferably, the API solvent is selected from methanol, isopropanol, ethanol and acetonitrile and mixtures thereof and more preferably is methanol.

Advantageously, the first solution comprises between 1 and 3 mg/ml of API, preferably between 2 and 3 mg/ml of API, even preferably between 1.3 and 1.8 mg/ml of API.

### Step (ii)

In the present invention, the term "API antisolvent" means a solvent or a mixture of solvents which dissolves less API than the API solvent or do not dissolve the API, while being miscible with the API solvent used in the preparation. Advantageously, the API antisolvent may be chosen as sufficiently volatile to be removed if necessary. Advantageously, the API antisolvent may be chosen as to being miscible with the API solvent used in the preparation.

For example, the API antisolvent may be chosen among water, an aqueous solution of glucose, sucrose, lactose, trehalose, NaCl, KCl, Na₂HPO₄, and /or KH₂PO₄, SH Buffer (300 mM sucrose, 20 mM HEPES, pH 7.4), HBS Buffer (150 mM NaCl, 20 mM HEPES, pH 7.4), PBS Buffer (137 mM NaCl, 2.7 mM KCl, 10 mM Na₂HPO₄, 1.8 mM KH₂PO₄, pH 7.4), and mixtures thereof. When the API is podophyllotoxin and podophyllotoxin derivatives such as etoposide and teniposide, the preferred API antisolvent is water.

Unexpectedly, the inventors have found that when the stabilizing agent P1 to API weight ratio is below a certain threshold, the nanosuspension resulting from the redispersion of this nanostructured powder contained nanocrystals whose size is significantly reduced. Suitable stabilizing agents P1 may be selected from stabilizing agents mentioned above. Advantageously, stabilizing P1 is a poloxamer, more advantageously Poloxamer 407. Therefore, in the present invention, the method according to the first object of the invention uses a stabilizing agent P1 in a stabilizing agent P1 to API weight ratio equal or less than 5. Advantageously, the third mixture which is a miscible liquid mixture has a stabilizing agent P1 to API weight ratio equal or less than 4, equal or less than 3, equal or less than 2, equal or less than 1, equal or less than 0.5, equal or less than 0.2.

Advantageously, the second solution comprises between 0.01 g/100 mL and 0.3 g/100 mL of stabilizing agent, more advantageously between 0.01g/100 mL and 0,2 g/100 mL of stabilizing agent.

Even more unexpectedly, the inventors have found that nanocrystals may be obtained when no stabilizing agent P1 is used. Thus, advantageously, the method according to the first object of the invention does not use any stabilizing agent P1, thereby increasing the yields of API available for step iii).

Advantageously, mixing step (ii) is carried out by injection of the first solution into the second solution or by injection of the second solution into the first solution.

The volumes of API antisolvent and API solvent should be such as to allow the precipitation of the API. Interestingly, the inventors have shown that the size of nanocrystals diminishes when the ratio of the API antisolvent to API solvent increases. Advantageously, the API antisolvent volume to API solvent volume ratio is at least of 4.5, preferably at least of 5, at least of 5.5, at least of 6, at least of 6.5, more advantageously at least of 10.

More advantageously, in the method for manufacturing a nanostructured powder of the invention, the stabilizing agent P1 to API weight ratio is equal or less than 5 preferably equal or less than 4, less than 3, less than 2, less than 1 and the API antisolvent volume to API solvent volume ratio is at least of 4.5, preferably at least of 5, at least of 5.5, at least of 6, at least of 6.5 more preferably at least of 10.

Even more advantageously, in the method for manufacturing a nanostructured powder of the invention, the stabilizing agent P1 to API weight ratio is equal or less than 4 and the API antisolvent volume to API solvent volume ratio is at least of 10.

### Step (iii)

When the first solution and the second solution are mixed, API is possibly precipitated and the third mixture as disclosed can therefore be regarded as a suspension. In this case, the suspension obtained at the end of step (ii) may comprise particles having a size inferior to 500 nm, preferably a size inferior to 250 nm, more preferably a size inferior to 100 nm, even more preferably a size inferior to 50 nm.

When the first solution and the second solution are mixed, API is not precipitated and the third mixture of the invention can therefore be regarded as a miscible liquid mixture containing the API solvent, the API antisolvent, and solubilized API. In other terms, the liquid mixture obtained at the end of step (ii) does not comprise any nanoparticles or microparticles. Advantageously, such a mixture allows API solubilization in a solvent/antisolvent phase. Advantageously, the API is solubilised in the API solvent.

The absence of nanoparticles or microparticles at the end of step (ii) in the embodiments described above is particularly advantageous, as steps (ii) and (iii) render superfluous an intermediate step between step (ii) and (iii) intended to eliminate nanometric and/or micrometric particles. In other terms, the process does not comprise any intermediate step between steps (ii) and (iii); in particular any filtration or any other step intended to eliminate micrometric particles. This is particularly advantageous in terms of API yield, as it avoids to eliminate the API incorporated in said particles.

The API nanocrystals form and/or develop during step (iii).

Step (iii) may be carried out under vacuum, for example at a pressure below 15 mbar, preferably below 20 mbar. A rotary evaporator may be used. Advantageously, step (iii) may be carried out at room temperature, typically between 10°C and 25°C.

Preferably, the evaporation step (iii) is carried out under vacuum, preferentially using a rotary evaporator. In an embodiment, the evaporation is carried at ambient temperature (e.g. 15° to 25°C). In another embodiment, the evaporation is carried out with the application of a moderate heat elevating the temperature of the suspension obtained in step (ii) to a temperature range of between 20 and 80°C, preferably between 20 and 40°C, more preferably between 20 and 30°C.

Advantageously, the evaporation step (iii) is carried out until complete removal of both API solvent (which is typically an organic solvent) and API antisolvent (which is typically water) from the suspension of step (ii). In this case, the nanostructured powder obtained at the end of step (iii) is free of API solvent (e.g. organic solvent such as methanol) and API antisolvent (e.g. water).

The nanostructured powder obtained with the method according to the first object of the invention is defined in more detail below.

### Nanostructured powder

A second object of the invention is a nanostructured powder comprising nanocrystalline agglomerates containing API in crystalline form, said agglomerates having at least one dimension of less than 1 µm.

The nanostructured powder of the invention is able to be obtained by the method of the first object of the invention.

Advantageously, in the present invention, the term "powder" means an assembly of discrete particles, each particle having a mean size usually less than 100 µm. Advantageously, the nanostructured powder is free of organic solvent and/or water. The term "agglomerate" means an assembly of particles loosely bonded and easily dispersible. The term "nanocrystalline agglomerate" refers to an agglomerate that, when dispersed, gives rise to nanocrystals.

Thus, the term "agglomerates containing API in crystalline form" denotes agglomerates whose chemical composition at the scale of the agglomerate is identical from one agglomerate to another, each of the agglomerate representing an assemblage (or a set) of API nanocrystals loosely bonded and easily dispersible, wherein the API constituting the powder of the invention is crystalline, i.e. it is not amorphous, or that its X-ray diffraction pattern has a crystalline signature. In other words, its X-ray diffraction pattern shows the presence of diffraction peaks.

Advantageously, the agglomerates of the nanostructured powder of the invention have a polyhedral form and at least one dimension of less than 1 µm.

Advantageously, the agglomerates have a mean size which ranging from about 1 to 20 µm, and more preferably ranging from about 1 to 10 µm, even more advantageously ranging from 1 to 5 µm.

The size distribution of the agglomerates may be measured by scanning electron microscopy (SEM), notably with equipment marketed under the reference Philips XL 30 microscope.

In an embodiment, the nanostructured powder of the invention consists essentially of crystalized API. In other terms, the nanostructured powder consists of at least 95 %wt., 96 %wt., 97 %wt., 98 %wt., 99 %wt., 99.5 %wt., 99.6 %wt., 99.7 %wt., 99.8 %wt. or 99.9 %wt. API, the remainder being inevitable impurities resulting from the method for preparing said nanostructured powder such as the one according to the first object of the invention. Such nanostructured powder consisting essentially of API may advantageously be obtained when no stabilizing agent P1 is used in the nanostructured powder preparation method according to the first object of the invention. Preferably, the nanostructured powder consists of 100 %wt. of API.

In another embodiment, the nanostructured powder of the invention consists essentially of crystalized API and stabilizing agent. Advantageously, the nanostructured powder consists of at least 90 %wt., 95 %wt., 96 %wt., 97 %wt., 98 %wt., 99 %wt., 99.5 %wt., 99.6 %wt. 99.7 %wt., 99.8 %wt. or 99.9 %wt. API, the remainder being stabilizing agent and inevitable impurities resulting from the method for preparing said nanostructured powder such as the one according to the first object of the invention. Such nanostructured powder consisting essentially of API and stabilizing agent may be obtained by the method according to the first object of the invention. In this case, the stabilizing agent present in the nanostructured powder is stabilizing agent P1 in said method.

The inevitable impurities resulting from the method according to the first object of the invention may originate from the API source used in the nanostructured powder preparation method according to the first object of the invention. Other impurities may be API solvent (e.g. methanol), antisolvent (e.g. water) traces. Advantageously, the nanostructured powder of the invention is free of API solvent (e.g. organic solvent such as methanol) and/or API antisolvent (e.g. water), more advantageously is free of API solvent and of API antisolvent.

Advantageously, the nanostructured powder of the invention is free of API that has lost its original biological activity.

Advantageously, the nanostructured powder of the invention is free of contaminant typically produced during wet milling techniques i.e. residues of the milling balls made of ceramic (cerium or yttrium-stabilized zirconium oxide), stainless steel, glass, or highly cross-linked polystyrene resin-coated.

Advantageously, the nanosuspension according to the invention is stable for at least 10 days, more advantageously 20 days.

The nanostructured powder according to the invention may be formulated in solid dosage form, for example capsules, tablets, pills, powders, dragees or granules with the addition of binders and other excipients known in the art.

### Nanosuspension preparation

A third object of the invention is a method of manufacturing a nanosuspension of API nanocrystals comprising a step of mixing at least a nanostructured powder according to the second object of the invention and an API antisolvent, e.g., by means of a magnetic stirrer or any other rotating device, preferably with a speed of up to 1000 rpm.

Advantageously, the method comprises a step of mixing a nanostructured powder according to the second object of the invention, an API antisolvent and optionally a stabilizing agent P2 in a stabilizing agent P2 to API weight ratio equal or less than 5, preferably less than 2.

Suitable stabilizing agents P2 may be selected from the stabilizing agents mentioned above.

Advantageously, stabilizing agent P2 may be selected from polysorbate 80, polyvinylpyrrolidone, hydroxypropylmethylcellulose, sodium lauryl sulfate, mannitol, lecithin, tocopheryl polyethylene glycol 1000 succinate, sorbitan trioleate 85, tyloxapol, poloxamer 338, sodium cholate, cholic acid, leucine, distearoyl-sn-glycero-3-phosphoethanolamine-N-[amino(polyethylene glycol)-1000, distearoyl-sn-glycero-3-phosphoethanolamine-N-[amino(polyethylene glycol)-2000, distearoyl-sn-glycero-3-phosphoethanolamine-N-[amino(polyethylene glycol)-5000, vitamin E, tragacanth, decyl glucoside, fetal calf serum, soy phosphatidylcholine, egg phosphatidylcholine, a synthetic derivative of phophatidylcholine, dioctyl sulfosuccinate, chitosan, maltose, Asialofetuin, transferrin, albumin, cyclodextrine, poloxamer 188, and poloxamer 407 and mixtures thereof. Preferably, the stabilizing agent P2 is poloxamer 407.

Advantageously, the stabilizing agent P2 is present in the API antisolvent before mixing the nanostructured powder or added together with the nanostructured powder.

The mixing step may typically comprise a sonication or an agitation step to help nanostructured powder dispersion.

For the preparation of a nanosuspension according to the invention the API antisolvent is preferably a solvent or a mixture of solvents capable of dispersing the agglomerates of the nanostructured powder according to the second object of the invention. Preferably, the solvent is a pharmaceutically acceptable solvent or a mixture of pharmaceutically acceptable solvents.

For example, the API antisolvent used in the nanosuspension preparation may be water, preferably distilled water. The water used as solvent may be any kind of water, such as normal water, purified water, distilled water, bi- or tri-distilled water, or demineralized water. Accordingly, the resulting nanosuspension is an aqueous nanosuspension.

The nanostructured powder can be prepared as disclosed above.

### Nanosuspension

A fourth object of the invention is a nanosuspension comprising API nanocrystals and an API antisolvent, wherein the API nanocrystals have an average size of less than 500 nm.

The nanosuspension of the invention is able to be obtained by the method of the third object of the invention mentioned above. Thus, advantageously, the nanosuspension of the invention is obtainable by the method of the third object of the invention and comprises API nanocrystals and an API antisolvent, wherein the API nanocrystals have an average size of less than 500 nm.

Advantageously, the nanosuspension of the invention further comprises a stabilizing agent, the weight ratio of stabilizing agent to API is equal or inferior to 5, equal or inferior to 4, equal or inferior to 3, equal or inferior to 2, equal or inferior to 1, equal or inferior to 0.9, equal or inferior to 0.8, equal or inferior to 0.7, equal or inferior to 0.6, equal or inferior to 0.5, equal or inferior to 0.4, equal or inferior to 0.3, equal or inferior to 0.2, equal or inferior to 0.1.

Advantageously, such stabilizing agent may be selected from polysorbate 80, polyvinylpyrrolidone, hydroxypropylmethylcellulose, sodium lauryl sulfate, mannitol, lecithin, tocopheryl polyethylene glycol 1000 succinate, sorbitan trioleate 85, tyloxapol, poloxamer 338, sodium cholate, cholic acid, leucine, distearoyl-sn-glycero-3-phosphoethanolamine-N-[amino(polyethylene glycol)-1000, distearoyl-sn-glycero-3-phosphoethanolamine-N-[amino(polyethylene glycol)-2000, distearoyl-sn-glycero-3-phosphoethanolamine-N-[amino(polyethylene glycol)-5000, vitamin E, tragacanth, decyl glucoside, fetal calf serum, soy phosphatidylcholine, egg phosphatidylcholine, a synthetic derivative of phophatidylcholine, dioctyl sulfosuccinate, chitosan, maltose, Asialofetuin, transferrin, albumin, cyclodextrine, poloxamere 188, and poloxamere 407 and mixtures thereof. Preferably, the stabilizing agent is poloxamere 407.

In the present invention, the expression "nanocrystal" denotes a particle in the nanometer range whose chemical composition at the scale of the nanocrystal is identical from one nanocrystal to another, each of the nanocrystal representing an assemblage of more than thousands of API molecules that associate in a crystalline form. Thus, a nanocrystal is composed of a solid API core that does not contain stabilizing agents such as surfactant or polymers. This does not exclude that a nanocrystal may be surrounded by a layer of stabilizing agent.

Advantageously, the average size of the nanocrystals in a nanosuspension of the invention as measured by Dynamic Light Scattering, notably with equipment marketed under the reference Zetasizer Nano-ZS by the company Malvern Instrument, is less than 500 nm, 400 nm, 300 nm, 200 nm. More advantageously, the average size of the nanocrystals ranges between about 40 nm and 100 nm, preferably between 50 and 100 nm.

Advantageously, the nanocrystals have a PDI of about equal or inferior to 1, more advantageously to 0.9.

Advantageously, in particular when no stabilizing agent P1 is used in the preparation method according to the first object of the invention, the nanocrystals have a PDI is equal or inferior to 0.4, more advantageously 0.3.

In an embodiment, the nanocrystals of the invention consist essentially of crystalized API. In other terms, the nanocrystals consist of at least 95 %wt., 96 %wt., 97 %wt., 98 %wt., 99 %wt., 99.5 %wt., 99.6 %wt. 99.7 %wt., 99.8 %wt. or 99.9 %wt. API, the remainder being inevitable impurities resulting from the method for preparing said nanostructured powder according to the first object of the invention. Such NCs consisting essentially of API may advantageously be obtained when no stabilizing agent P1 is used in the nanostructured powder preparation method according to the first object of the invention. Preferably, the nanocrystals consist of 100 %wt. of API. In another embodiment, the nanocrystals of the invention consist essentially of crystalized API and stabilizing agent. In other terms, the nanocrystals consist of at least 90 %wt., 95 %wt., 96 %wt., 97 %wt., 98 %wt., 99 %wt., 99.5 %wt., 99.6 %wt. 99.7 %wt., 99.8 %wt. or 99.9 %wt. API, the remainder being stabilizing agent and inevitable impurities resulting from the method for preparing said nanostructured powder according to the first object of the invention. Such nanocrystals consisting essentially of API and stabilizing agent may be obtained by the method according to the third object of the invention, in which the nanostructured powder can be optionally obtained by the method according to the first object of the invention. In this case, the stabilizing agent present in the nanocrystals is stabilizing agent P1 when stabilizing agent P1 is used in the preparation method according to the first object of the invention and/or stabilizing agent P2 when stabilizing agent P2 is used in the preparation method according to the third object of the invention.

Advantageously, the nanosuspension of the invention is free of API that has lost its original biological activity.

Advantageously, the nanosuspension of the invention is free of contaminant typically produced during wet milling techniques i.e. residues of the milling balls made of ceramic (cerium or yttrium-stabilized zirconium oxide), stainless steel, glass, or highly cross-linked polystyrene resin-coated.

Advantageously, the nanosuspension according to the invention is stable for at least 6 hours, preferably at least 24 hours.

The nanosuspension according to the fourth object of the invention may be dried, e.g., by lyophilization, fluid or spray drying, into powders, which may be formulated in solid dosage form, for example capsules, tablets, pills, powders, dragees or granules with the addition of binders and other excipients known in the art of tablet making.

### Pharmaceutical uses

A fifth object of the invention is the nanosuspension according to the fourth object of the invention for its use as a medicament.

Advantageously, the nanosuspension of the invention may be administered by any convenient route including intravenous, oral, transdermal, intrapulmonary. Intravenous route is of particular interest.

In particular, if the API exhibit an anticancer activity, for example if the API is selected among podophyllotoxin and podophyllotoxin derivatives and mixtures thereof such as etoposide and teniposide, the nanosuspension of the invention may be particularly useful for treating cancer, more advantageously for treating a cancer selected from testicular cancer, lung cancer, lymphoma, leukemia, neuroblastoma, and ovarian cancer.

Further aspects and advantages of the present invention will be disclosed in the following experimental section, which should be regarded as illustrative and not limiting the scope of the present application.

### EXPERIMENTAL PART

The raw materials used in the examples are listed below:
- Commercial powder of etoposide, VP-16, Clinisciences, Purity 99.81%
- Etoposide chemotherapy medication, Toposar, TEVA
- Methanol, Methanol RS, Carlo Erba, Impurities < 5ppm
- Poloxamer P407, Pluronic F-127, BASF, Impurities 50-125 ppm butylated hydroxytoluene
- Poloxamer P188, Pluronic F-68, BASF, Impureties 50-125 ppm butylated hydroxytoluene

Unless stated otherwise, all the materials were used as received from the manufacturers.

The materials, prepared or commercial, were characterized by:
- Scanning electron microscopy (SEM) to observe the relative surface morphology and the structure of the API nanostructured powder;
- X-ray diffraction (XRD) to verify the crystalline nature of the API nanostructured powder;
- Differential Scanning Calorimetry experiments to observe the thermal behavior of the API e.g. pure API, pure processed API (nanocrystals), API NCs/Pluronic F-127 solid dispersion;
- Thermomicroscopy to observe properties of the API into solid state;
- Transmission electron microscopy (TEM) to evaluate the morphology of the nanostructured powder, and/or nanocrystalline agglomerates;
- Tunable Resistive Pulse Sensing (TRPS) qNano was used as a complementary method to evaluate the size of etoposide nanocrystals right after redispersion in solution.

### Example 1: Study of the relationship between the API/P1 weight ratio and API particle size in the corresponding API nanosuspensions.

### 1.1 Material and methods

Merely, a commercial powder of etoposide (ETO) as an API was dissolved in absolute methanol in glass vial and slowly injected under agitation (1200 rpm) in water containing P407 as P1 with different ETO/P1 weight ratios (see Table 1).

The mixture was then precipitated by evaporating the entire solution using a rotovapor under vacuum for 0.5 h. The resulting powder was kept under vacuum to remove any traces of methanol, then hydrated with aqueous solution containing various quantity of P407 as polymer P2 under 10 min sonication using a water-bath sonicator to engineer the nanocrystals dispersion.

The size of etoposide nanoparticles was then evaluated by DLS to explore the impact of the weight ratio of API/P1 on the size of ETO particles in the obtained etoposide nanosuspension.

### 1.2 Results

**Table 1: Size of ETO nanoparticles in suspension versus P407 addition during evaporation (as stabilizing agent P1) and after redispersion (as stabilizing agent P2) in water.**

| *Test* | *precipitation parameters* | | | | *Dispersion parameters* | | *ETO nanoparticles* | | | |
|---|---|---|---|---|---|---|---|---|---|---|
| | *ETO (mg)* | *MeOH (mL)* | *Water (mL)* | *Polymer P1 (mg)* | *Water (mL)* | *Polymer P2 (mg)* | *Size (nm) @t=0h* | *Size (nm) @t=6h* | *PDI* | *Counts (kpcs)* |
| *#1* | *2.5* | *1.5* | *10* | *11* | *6* | *0* | *24* | *91* | *0,413* | *129* |
| *#2* | *2.5* | *1.5* | *10* | *12* | *6* | *0* | *5* | *71* | *1* | *365* |
| *#3* | *2.5* | *1.5* | *10* | *15* | *6* | *0* | *376* | *464* | *0,285* | *157* |
| *#4* | *2.5* | *1.5* | *10* | *20* | *6* | *0* | *552* | *530* | *0,574* | *17626* |
| *#5* | *2.5* | *1.5* | *10* | *30* | *6* | *0* | *540* | *492* | *0,546* | *23840* |
| *#6* | *2.5* | *1.5* | *10* | *1* | *6* | *10* | *8* | *28* | *0,833* | *203* |
| *#7* | *2.5* | *1.5* | *10* | *2* | *6* | *10* | *5* | *66* | *0,888* | *259* |
| *#8* | *2.5* | *1.5* | *10* | *5* | *6* | *10* | *5,5* | *68* | *0,873* | *236* |
| *#9* | *2.5* | *1.5* | *10* | *10* | *6* | *10* | *5* | *100* | *1* | *233* |
| *#10* | *2.5* | *1.5* | *10* | *20* | *6* | *10* | *645* | *644* | *0,992* | *89576* |
| *#11* | *2.5* | *1.5* | *10* | *0* | *6* | *2* | *78* | *129* | *0.317* | *2231* |

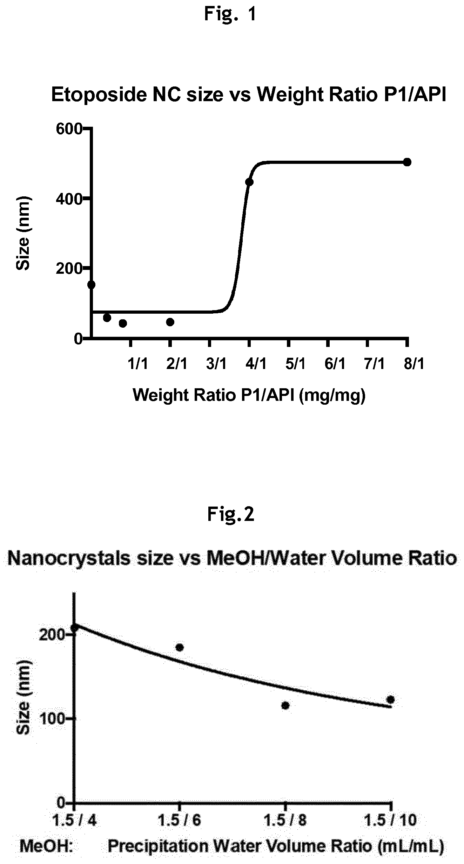

As shown in Table 1 and Figure 1, the amount of stabilizing agent added before evaporation (P1) influences the size of ETO nanocrystals (NCs) in suspension. In particular, when 10 mg or less of P407 for 2.5 mg total ETO is used, NCs of size of approximately 100 nm are obtained, contrary to the nanodispersion prepared with more P407, where NCs have a size ranging from 450 nm to 650 nm.

The results obtained for these precipitation processes show that in the liquid antisolvent bottom up method of the invention, there's no need adding a stabilizing agent in the aqueous solution for the evaporation step (stabilizing agent P1), but adding it only to the aqueous solution that redisperse the NCs after evaporation (stabilizing agent P2) is sufficient, which is of significant impact on yields of the drug.

Furthermore, data also show that the size of NCs is correlated with stabilizing agent P1/API weight ratio, a stabilizing agent P1 to API weight ratio equal or less than 5, even less than 4 lead to NCs around 100 nm in size.

Similar results regarding the size of NCs were obtained when using P188 as stabilizing agent P1 (not shown).

### Example 2: Study of the relationship between ETO solvent to precipitation water volume ratio and particle size in the corresponding ETO nanosuspensions.

### 2.1 material and methods

The protocol exposed in Example 1 was followed with the parameters shown in Table 2.

**Table 2: ETO nanosuspension manufactured by varying methanol: precipitation water volume ratio.**

| *Test* | *precipitation parameters* | | | | *Dispersion parameters* | |
|---|---|---|---|---|---|---|
| | *ETO (mg)* | *MeOH (mL)* | *Water (mL)* | *Polymer P1 (mg)* | *Water (mL)* | *Polymer P2 (mg)* |
| *15#* | *2.5* | *1.5* | *4* | *0* | *6* | *5* |
| *16#* | *2.5* | *1.5* | *6* | *0* | *6* | *5* |
| *17#* | *2.5* | *1.5* | *8* | *0* | *6* | *5* |
| *18#* | *2.5* | *1.5* | *10* | *0* | *6* | *5* |

### 2.2 results

As shown in Figure 2, an increase of ETO nanoparticles size is observed with a diminution of the methanol: precipitation water volume ratio. This may be explained by the fact that the more the etoposide is diluted, the more its dispersion is fostered. For the further experiments, a methanol to precipitation water volume ratio of 1:10 was applied to produce the etoposide NCs powder.

### Example 3: Study of the relationship between the nature and concentration of P2 polymer and ETO particle size evolution in the corresponding ETO nanosuspensions.

### 3.1 Material and methods

The protocol exposed in Example 1 was followed, except that etoposide was solubilized in methanol and then injected in water without stabilizing agent P1, followed by a complete evaporation and redispersion in water with 0.5 mg or 2 mg of Pluronic F-127 or Pluronic F-68 as stabilizing agent P2.

The size of etoposide nanoparticles was then evaluated by DLS to explore the impact of the nature and concentration of P2 on the nanocrystal size.

### 3.2 Results

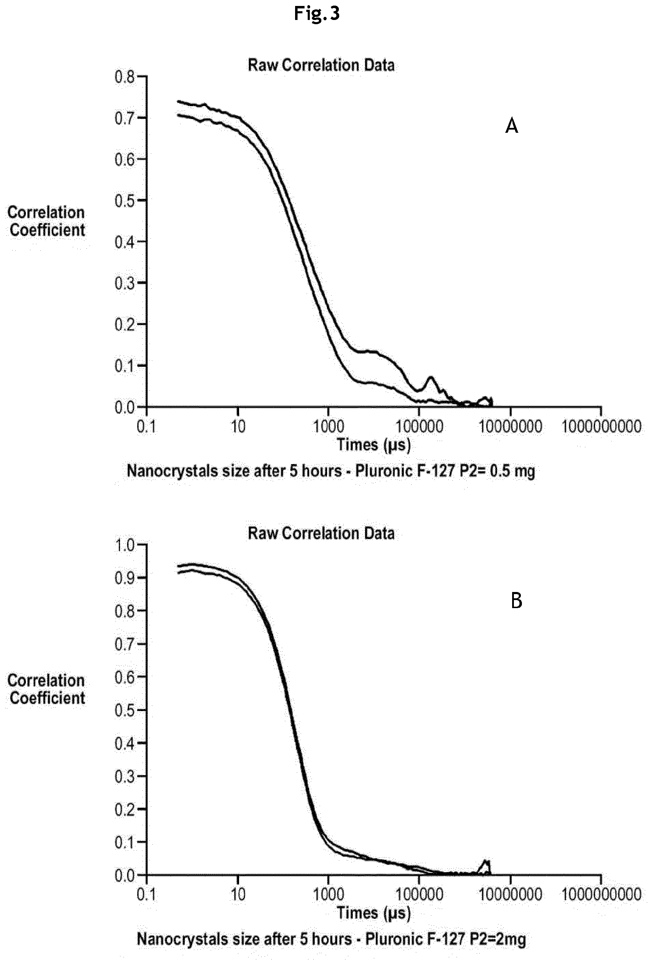

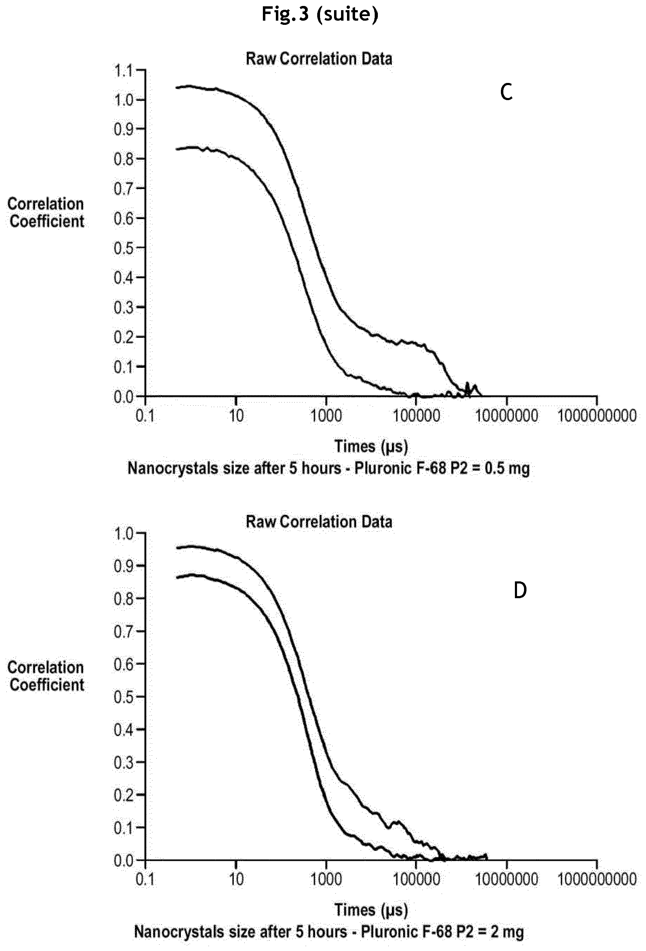

The shape of the correlograms show that the nanocrystals are better stabilized with 2 mg of P2 stabilizing agent as regard to 0.5 mg, for both Pluronic F-127 (Figures 3A and 3B) and Pluronic F-68 (Figures 3C and 3D). Moreover, the stability of NCs is found better with Pluronic F-127 as its affinity (*via* e.g. hydrophobic/hydrophilic or van der Waals interactions) as compared to the Pluronic F-68. This property is confirmed with the overlapping of the two correlograms in the case of the etoposide NCs stabilized with Pluronic F-127.

As can be seen on Table 3, The nanodispersion prepared with 2 mg F-127 (#11) presents a size of approximately 120 nm after 5 h, and 150 nm at 24h. The nanodispersion prepared with F188 (#13) has a size of 240 nm after 6 h, however the nanodispersion precipitated after 24h revealing that nanocrystals were not fully stabilized with Pluronic F-68. The results obtained led to preferentially use Pluronic F-127 when no stabilizing agent P1 is used, even if F-68 leads to nanoparticles in the nanosize range.

**Table 3: Size of ETO nanoparticles in suspension versus F-127 or F-68 addition after redispersion (stabilizing agent P2) in water.**

| *Test* | *precipitation parameters* | | | | *Dispersion parameters* | | *ETO nanoparticles* | | | |
|---|---|---|---|---|---|---|---|---|---|---|
| | *ETO (mg)* | *MeOH (mL)* | *Water (mL)* | *Polymer P1 (mg)* | *Water (mL)* | *Polymer P2 (mg)* | *Size (nm) @t*=*0h* | *Size (nm) @t*=*5h* | *Size (nm) @t*=*24h* | *Counts (kpcs)* |
| *#11* | *2.5* | *1.5* | *10* | *0* | *6* | *2 mg* | *78* | *129* | *142* | *2231* |
| | | | | | | *F-127* | | | | |
| *#12* | *2.5* | *1.5* | *10* | *0* | *6* | *0.5 mg* | *110* | *282* | *300* | *290* |
| | | | | | | *F-127* | | | | |
| *#13* | *2.5* | *1.5* | *10* | *0* | *6* | *2 mg* | *205* | *242* | *Precipitation* | *245* |
| | | | | | | *F-68* | | | | |
| *#14* | *2.5* | *1.5* | *10* | *0* | *6* | *0.5 mg* | *117* | *337* | *Precipitation* | *178* |
| | | | | | | *F-68* | | | | |

### Example 4: Comparison of the morphology of ETO nanostructured powder according to the invention and marketed ETO powder

### 4.1 Material and methods

A pure commercial etoposide powder and an etoposide nanostructured powder according to the invention were characterized by scanning electron microscopy (SEM) (Philips XL 30 microscope, Hillsboro, USA). The etoposide nanostructured powder was prepared according to the protocol described in Example 1 using 2.5 mg etoposide solubilized in 1.5 mL methanol injected in 10 mL of water without stabilizing agent P1, then completely evaporated. The powders were placed on a double-sided tape, then coated with a 30 nm layer of gold under vacuum (10-6 Pa) for 2 minutes, then observed using SEM at an accelerating voltage of 15 kV under vacuum.

### 4.2 Results

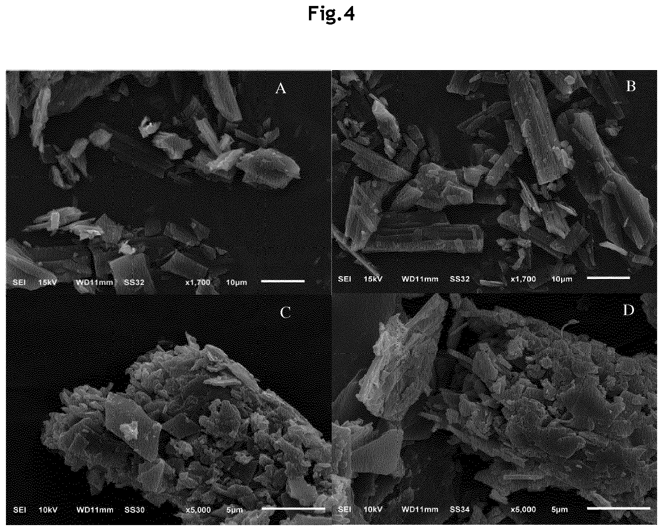

SEM experiments were performed to observe the relative surface morphology and the structure of the etoposide nanostructured powder according to the invention as compared to the pure commercial etoposide powder. The photographs clearly evidence a difference between the pure commercial etoposide powder (Figures 4A and 4B) and the etoposide nanostructured powder according to the invention (Figures 4C and 4D). Pure commercial etoposide powder comprises etoposide particles having mainly a rod shape form and a particle size for most of the particles above 1 µm while in the etoposide nanostructured powder according to the invention, agglomerates of a polyhedral shape with a mean size under 1 µm are observed. Moreover, such agglomerates have at least one dimension of less than 1 µm. Evident agglomeration of nanocrystals is observed (Figures 4C and 4D) before redispersion in water.

### Example 5: Comparison of the XRD patterns of ETO nanostructured powder and marketed ETO powder

### 5.1 Material and methods

The powder X-ray diffraction (PRDX) examinations of etoposide nanostructured powder according to the invention manufactured without using stabilizing agent P1 and commercial etoposide powder were made using a Bruker D8-Advance X-ray diffractometer equipped with a LynxEye silicon strip detector. The etoposide nanostructured powder was prepared according to the protocol described in Example 1 using 2.5 mg etoposide solubilized in 1.5 mL methanol injected in 10 mL of water without stabilizing agent P1, then completely evaporated. A copper source was used with a nickel filter leaving CuK_{α} radiation. The generator was set at 40 kV and 40 mA. The samples were ground and put in shallow-well sample holders. The results were collected as three frames to detect 2*θ* from 5 to 60 deg. for 300 seconds exposure and evaluated using the Bruker AXS and EVA softwares.

### 5.2 Results

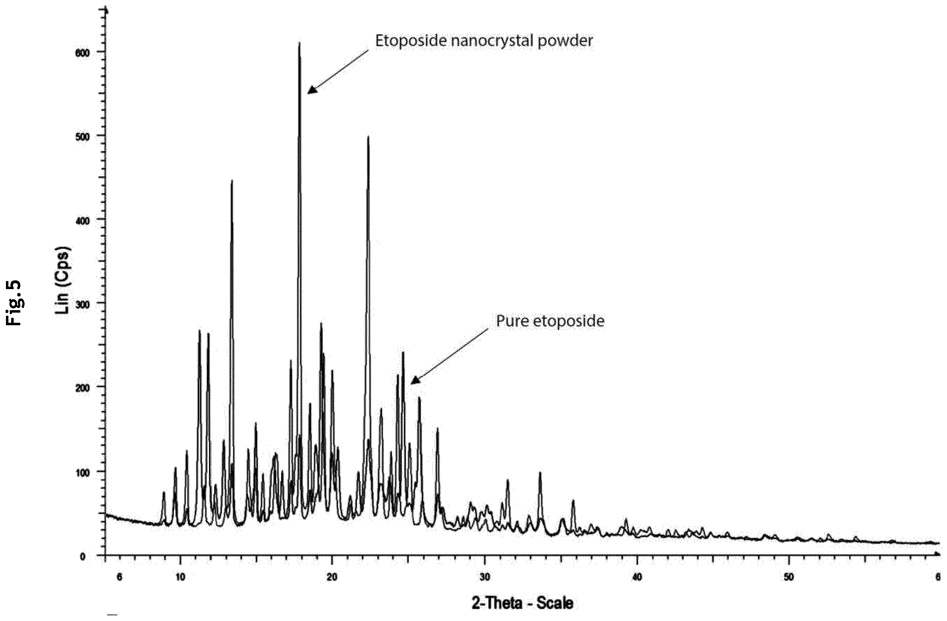

As shown in Figure 5, The X-ray diffraction pattern obtained for etoposide nanostructured powder confirms the total crystallinity of the etoposide after once the method of the invention implemented. However small shifts are observed for the etoposide nanocrystal powder compared to the marketed etoposide powder. This could be explained by a partial formation of another crystalline form (polymorph) of etoposide.

### Example 6: Comparison of the thermal behavior of ETO nanostructured powder according to the invention and marketed ETO powder

### 6.1 Material and methods

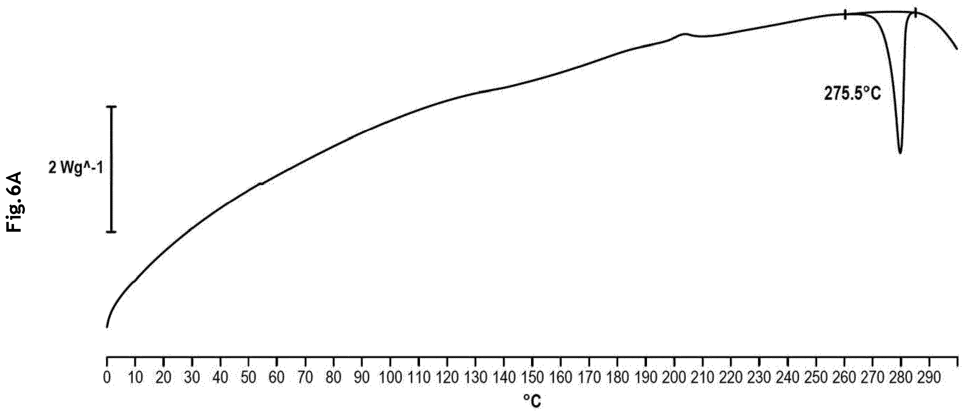

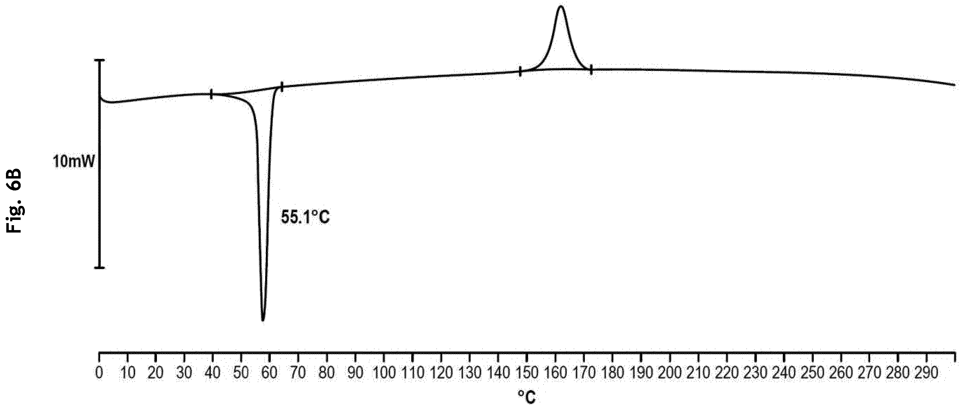

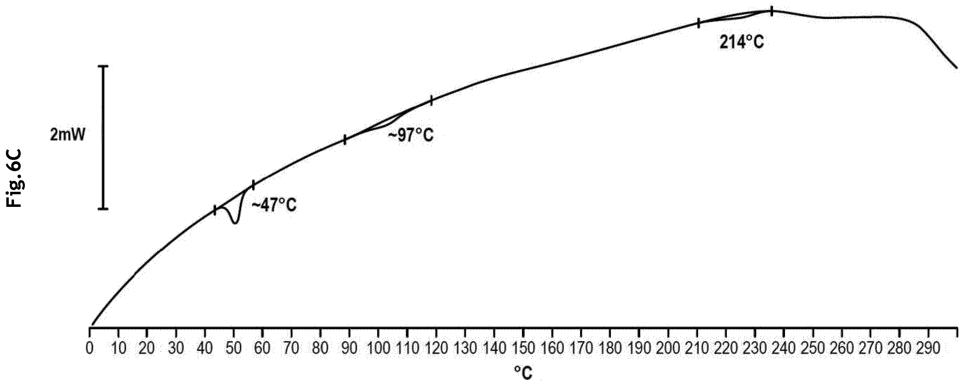

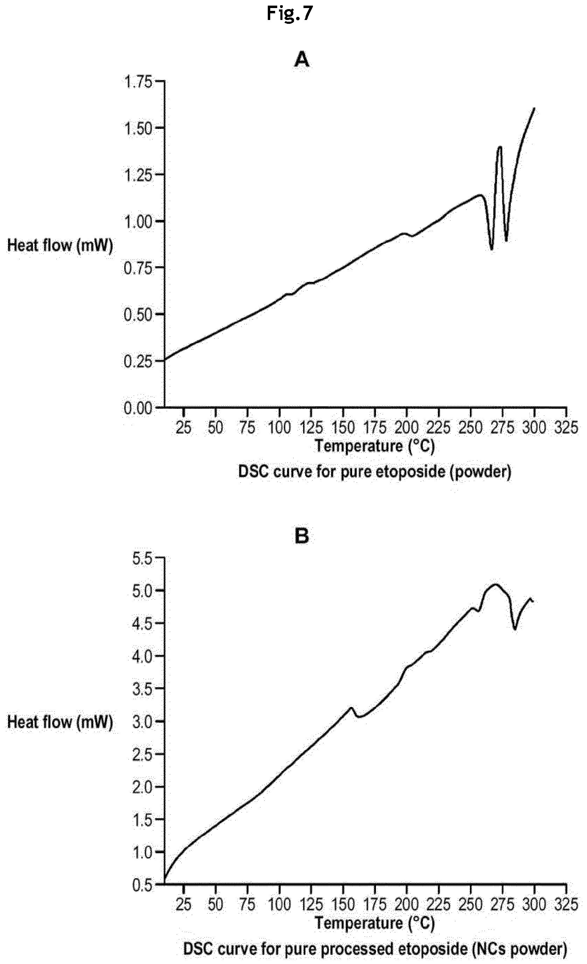

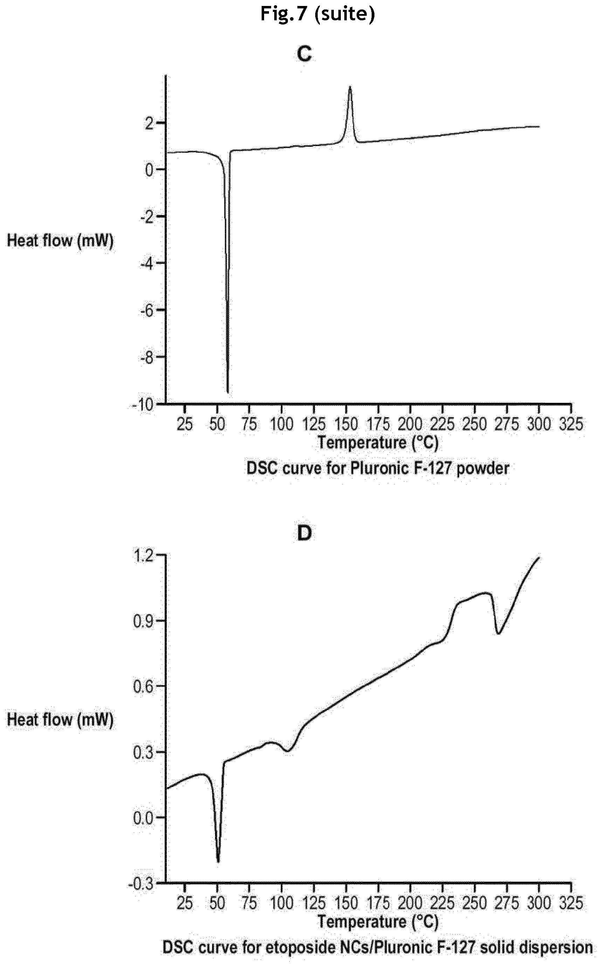

The thermal behavior of pure marketed etoposide powder, etoposide nanostructured powder according to the invention manufactured without using stabilizing agent P1, etoposide NCs/Pluronic F-127 0.03% wt/v dried solid dispersion (#11) and pure Pluronic F-127 were analyzed by differential scanning calorimetry (DSC) technique using a DSC3 from Mettler-Toledo (Greifensee, Switzerland). The etoposide nanostructured powder was prepared according to the protocol described in Example 1 using 2.5 mg etoposide solubilized in 1.5 mL methanol injected in 10 mL of water without stabilizing agent P1, then completely evaporated. Each sample with a known mass has been introduced in an aluminum pan that has been sealed afterward. An empty aluminum pan was used as reference. All experiments were performed in the temperature range from 0 to 300 °C with increments of 10 °C/min (Figure 6) or 5 °C/min (Figure 7) under a 50 mL/min dry that absorbs energy (endothermic transformation).

### 6.2 Results

The DSC curve of pure marketed etoposide powder exhibits an endothermic peak with an onset temperature of 275.5°C at a 10 °C/min scan rate (Figure 6A). This signal corresponds to the fusion of the compound. When the scan rate is reduced to 5 °C/min, one can observe a different behavior of etoposide upon melting with two endothermic peaks, indicating a thermal degradation of the etoposide during, or at least after its melting (Figure 7A). The DSC curve of nanostructured powder according to the invention manufactured without using stabilizing agent P1 exhibits two endothermic peaks with a precocious fusion peak at 250 °C (Figure 7B). This can be explained by the size reduction of etoposide nanocrystals that shift the melting temperature to a lower temperature. Nevertheless, the degradation signal of NCs etoposide takes place at the same temperature as that of pure etoposide at 5 °C/min (Figures 7A-7B). As far as the thermal behavior of etoposide NCs/Pluronic F-127 solid dispersion is concerned, the corresponding DSC curve presents a melting peak at about 47 °C corresponding to the fusion of Pluronic F-127 (*cf.* Figure 7C) and two other endothermic transformations (at about 97 °C and about 214 °C, *cf.* Figure 6C).

The depletion of 8 °C of the melting temperature of Pluronic F-127 in the solid dispersion (Figure 6C) compared to that of the pure polymer (Figure 6B) evidences the API/excipient interactions.

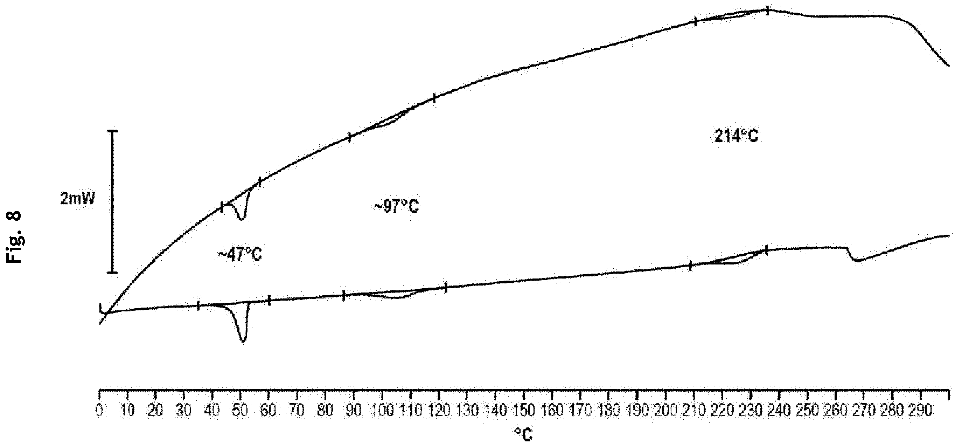

At this stage, the signal obtained for the solid dispersion around 97 °C cannot be explained, but interestingly, if this signal is independent of the scan rate (Figures 7D and 8), and ii/ has the same mass normalized energy with at least 3 different experiments, confirming the fact that if no degradation occurs at this temperature, and ii/ the solid dispersion formulations prepared as mentioned here are reproducible and homogeneous (*cf.* Figure 8).

The signal around 214 °C obtained for the etoposide NCs/Pluronic F-127 solid dispersion confirms the nanosized etoposide distribution within the polymeric matrix since the temperature of the corresponding signal is lower than that of pure processed etoposide (NCs).

Interestingly, the 3 main signals obtained for the etoposide NCs/Pluronic F-127 solid dispersion are reproducible and are not scan rate dependent, contrary to the forth one observed around 250-270 °C that is due to degradation of the solubilized etoposide in molten Pluronic F-127 (Figure 8).

### Example 7: Confirmation of the solid state of etoposide NCs when embedded in F-127 polymetric matrix

### 7.1 Material and methods

Etoposide NCs/Pluronic F-127 solid dispersions were observed as function of the temperature by means of a LTS 420 Linkam heating cell (Microvision Instruments, Evry, France) placed under a SMZ 168 microscope (Motic, Kowloon, Hong Kong). The etoposide NCs/Pluronic F-127 solid dispersion was prepared according to the protocol described in Example 1 using 2.5 mg etoposide solubilized in 1.5 mL methanol injected in 10 mL of water without stabilizing agent P1, then completely evaporated. Followed by a redispersion in water containing 0.03% wt/v F-127, and then completely evaporated. Temperature ranges from 23 to 300 °C at a 5 °C/min increment rate. Cooling of the system was achieved using a T95-HS Linkam device with liquid nitrogen automatically flowed through the cell. The pictures were taken each 5/12 °C (~ 0.42 °C) with a Moticam 2500, 5.0M pixels, from Motic.

### 7.2 Results

Thermal microscopy examination confirmed the solid state of etoposide NCs in the Pluronic F-127 polymeric matrix.

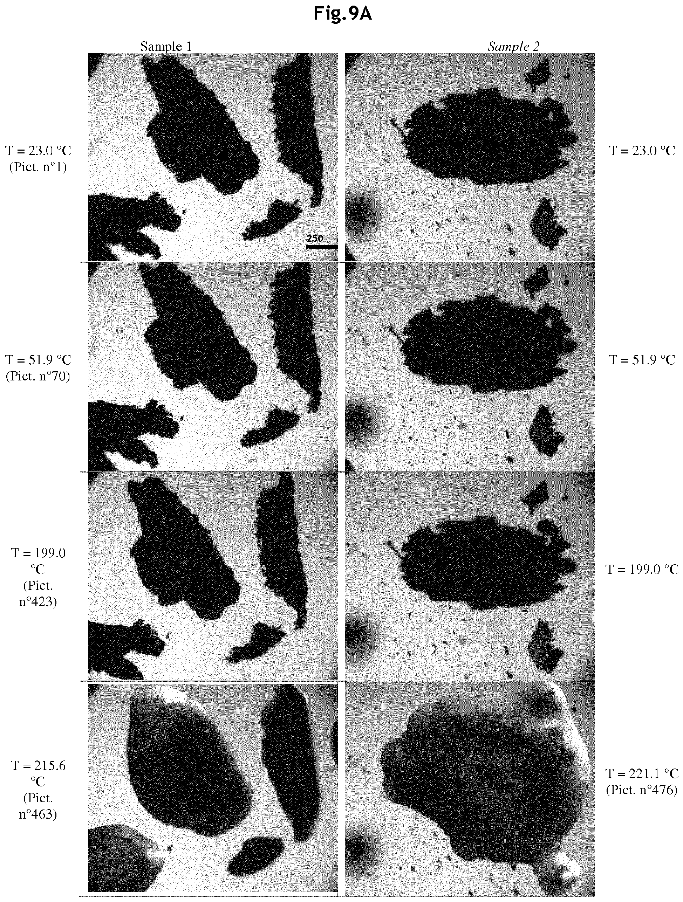

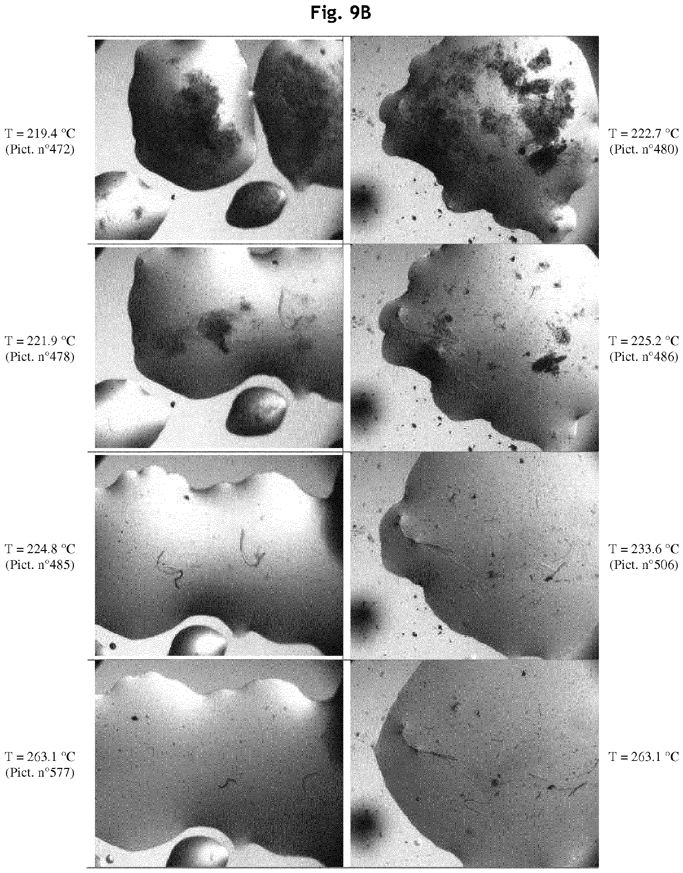

As it can be seen on Figure 9, the solid form of etoposide NCs in the F-127 polymeric matrix is clearly evidenced once the latter has melted (Pict. n°463-478 and n°476-486 for Sample 1, and Sample 2, respectively). Etoposide "fusion" in the F-127 molten system (*i.e.* etoposide dissolution) is observed at - 225.2°C which is good agreements with the above DSC results.

Degradation of the ETO dissolved in the molten Etoposide NCs/Pluronic F-127 solid dispersions can be observed after the dissolution process at - 263 °C (brown coloration, Pictures n°577 of Sample 1 and Sample 2), confirming the DSC results described above.

### Example 8: Comparison of the in vitro dissolution rate of an ETO nanostructured powder according to the invention and marketed ETO powder

### 8.1 Material and methods

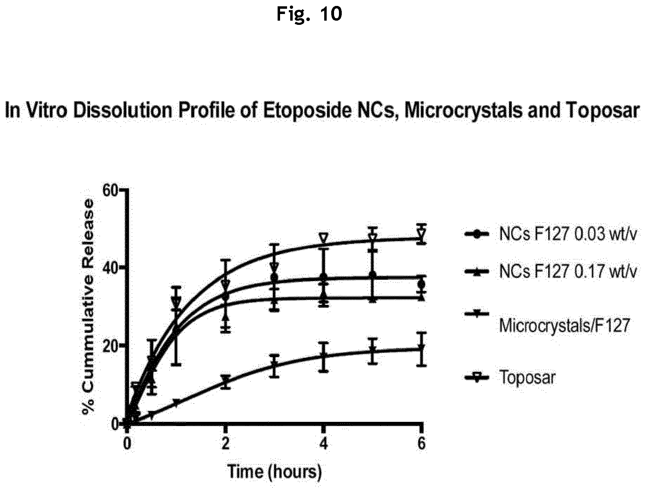

*In vitro* dissolution (Figure 10) released analysis was performed to compare the dissolution rate of etoposide NCs/Pluronic F-127 0.03% and 0.17% wt/v of the invention with the microcrystals from VP-16 powder dispersed in water with 0.03 wt% volume and sonicated for 5 minutes, of ETO in Toposar^{®} formulation in which ETO is in its solubilized state.

*In vitro* release of nanocrystals etoposide was assessed by the dialysis bag diffusion technique. The nanocrystals etoposide solution was placed in a cellulose dialysis bag (molecular weight cutoff 12.4 kDa) and sealed at both ends using dialysis tubing closure. The dialysis bag was placed in a compartment containing 40 mL of PBS-buffered saline medium, pH 7.4, which was stirred at 60 rpm and maintained at 37 °C for 6 hours. The receptor compartment was covered to prevent the evaporation of the continuum medium. Aliquots (1 mL) were withdrawn at 10 min, 30 min, 1, 2, 4, 6 hours, and the same volume of fresh PBS was added to the medium in order to maintain its overall volume at 40 mL after each sampling smear. Then, the samples were analyzed using a Cary 100 Scan UV-visible spectrophotometer (Pittsburgh, USA) set at 283 nm.

### 8.2 Results

Dissolution study of etoposide NCs evidenced the sustained release of the nanocrystalline forms of the invention compared to the marketed product (Toposar^{®}) and also showed an increase of the dissolution rate with the size reduction of particles (Figure 10). The etoposide NCs dispersion profiles show an increase of the dissolution rate as the specific area is more consequent for this solid dispersion compared to the etoposide microcrystals solid dispersion that has a low specific area (as particles size is larger) and therefore a lower dissolution rate. Only 18 % of initial mass has been released after 6 hours, whereas, for the nanocrystals formulation 35 % were released. Obviously, for the marketed product (Toposar^{®}), 50 % of etoposide were already released after 6 hours, as the etoposide is solubilized in 33% of ethanol for this formulation.

### Example 9: Comparison of the in vitro cytotoxicity of ETO nanostructured powder and marketed ETO powder

### 9.1 Material and methods

### 9.1.a. In vitro studies on CT26 cells only

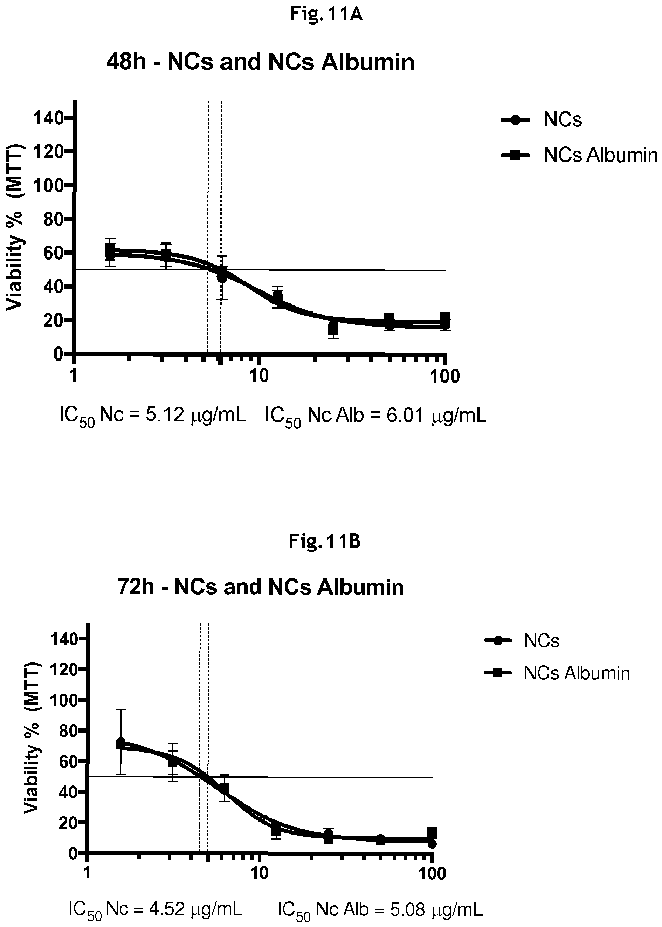

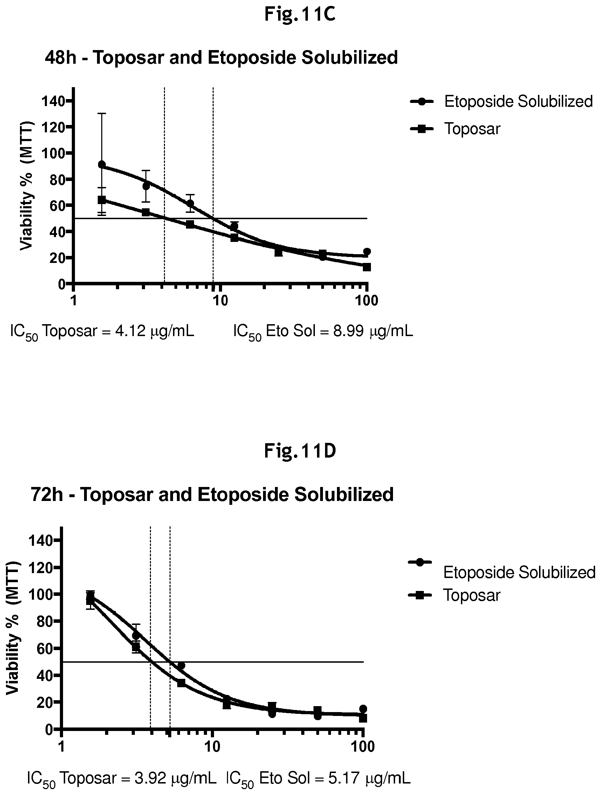

*In vitro* cytotoxicity studies for Etoposide NCs of the invention, F-127 alone, solubilized Etoposide and Toposar^{®} were performed on CT26 colon cancer cells. First, CT26 colon carcinoma cells were cultured in Dubelcco's modified Eagle's medium (DMEM) containing 10% foetal bovine serum and streptomycin (50 mmol) at 37 °C. Cells were plated at the concentration of 200,000 cells/mL in 96 well plates for 24 h. Then, CT26 cells were incubated with Etoposide NCs with or without albumin, solubilized Etoposide or Toposar^{®}. After 48 h or 72 h the tested formulations were removed from wells and cells viability assay was performed using the colorimetric MTT test, absorbance was determined at 562 nm in a microplate reader (BioKinetics Reader, EL340). The results of Figure 11 are displayed as percentage of viable cells.

### 9. 1.b In vitro studies on CT26 cells and LLC1 cells

Further tests were carried using the same protocol, this time with CT26 colon cancer and LLC1 Lewis lung cancer cells. Results are presented in 9.2.b below.

### 9.2 Results

### 9.2.a. In vitro studies on CT26 cells only

The viability of CT26 cells has been evaluated after 48h and 72h. Results are presented on Figure 11 and are summarized in Table 4 below. The etoposide NCs show similar results with and without albumin after 48h and 72h. The IC50 after 48h of etoposide NCs with and without albumin were 5.12 and 6.01 µg/mL, respectively, and 4.52 and 5.08 µg/mL at 72h. The additional coating of albumin did not significantly improve the sustained release and cytotoxicity of etoposide. However, the cytotoxicity of etoposide NCs of the invention outperformed the solubilized form, for which the IC50 at 48h was 8.99 µg/mL. Besides, cytotoxicity of etoposide NCs of the invention that are safer, as no alcohol and unwanted agent are used, can also compete with the marketed product (Toposar^{®}) were the IC50 at 48 h and 72h were 4.12 and 3.92 µg/mL respectively.

**Table 4: CT26 colon cancer cells viability after 48 and 72 hours incubation with etoposide nanocrystals with and without albumin or incubation with Toposar.**

| Formulation | IC 50 (48h) (µg/mL) | IC 50 (72h) (µg/mL) |
|---|---|---|
| Etoposide NCs | 5.12 | 4.52 |
| Etoposide NCs/Pluronic F-127 0.083% wt/v + Albumin 0.2% wt/v | 6.01 | 5.08 |
| Etoposide Solubilized | 8.99 | 5.17 |
| Toposar^{®} | 4.12 | 3.92 |

### 9.2.b. In vitro studies CT26 cells and LLC cells

Results of 9.1.b. are summarized in Table 5 below. The IC50 after 48h of ETO NCs on CT26 cells with and without albumin were 13.73 ± 5.52 and 20.06 ± 6.09 µM, respectively, and 4.66 ± 0.91 and 4.40 ± 1.20 µM at 72h. Hence, the ETO NCs show similar results with and without albumin after 48h (p > 0.05) and 72h (p > 0.05); and proved their efficiency to inhibited cell growth. The additional coating of albumin did not significantly improve the sustained release and cytotoxicity of ETO. However, the cytotoxicity of ETO NCs slightly outperformed the Free ETO, for which the IC50 at 72h was 5.60 ± 0.10 µM. Essentially, ETO NCs are safer, as no alcohol and undesired excipient are used and hence can compete with the marketed product Toposar^{®} were the IC50 at 48h and 72h were 16.71 ± 9.95 and 4.55 ± 0.50 µM respectively. Regarding 3LL cells line, all formulations tested were notably more efficient (p < 0.05) in comparison with the IC50 obtained for the CT26 cell lines.

**Table 5: CT26 colon cancer cells and LLC1 lung cancer cells viability after 48- and 72-hours incubation with etoposide nanocrystals with and without albumin or incubation with Toposar^{®}.**

| Formulation | CT26 - IC50 (48h) (µM) | CT26- IC50 (72h) (µM) | LLC1 - IC 50 (48h) (µM) | LLC1 - IC 50 (72h) (µM) |
|---|---|---|---|---|
| Etoposide NCs/ F-127 0.083% wt/v | 13.73 ± 5.52 | 4.66 ± 0.91 | 1.17 ± 0.12 | 1.01 ± 0.24 |
| Etoposide NCs/ F-127 0.083% wt/v + Albumin 0.2% wt/v | 20.06 ± 6.09 | 4.40 ± 1.20 | 1.31 ± 0.26 | 1.00 ± 0.20 |
| Free Etoposide | 11.28 ± 1.40 | 5.60 ± 0.10 | 1.66 ± 0.13 | 1.05 ± 0.02 |
| Toposar^{®} | 16.71 ± 9.95 | 4.55 ± 0.50 | 1.43 ± 0.13 | 0.75 ± 0.10 |

### Example 10: Nanocrystals cellular uptake CT26

### 10.1 Material and methods

In vitro TEM imaging studies of the invention were performed to observe the internalization of the Etoposide nanocrystals inside the cells. ETO NCs/ F-127 0.083% wt/v, ETO NCs/F-127 0.083 wt/v + albumin 0.2% wt/v were tested on CT26 and 3LL cancer cells. Cells (2.10⁵ cells/mL) were put in 25 cm³ culture flask for 24h until confluence. Then, CT26 cells were incubated with each ETO NCs formulations for only 2h. After this period, cells were trypsinized (Trypsin-EDTA 0.5%) and recovered in falcon^{®} tubes. Cells were water washed and fixed with paraformaldehyde 2% + glutaraldehyde 2.5% + Na cacodylate 0.1 M (pH 7.3) + CaCl₂ 5mM, samples were postfixed in 1% OsO₄ and stained with filtered (0.22 µm) uranyl acetate 1%. Then, specimens were rinsed in 0.1M phosphate buffer and dehydrated in an escalating streak of ethanol at 30, 50, 70, 95 and 100% (3 × 10 min for each) and passed on with propylene oxide and ethanol (50:50 v/v mixture) for 10 min. Followed by polymerization in Epon at 60°C for 72h. Samples were sliced (80 nm) using a Leica ultracut 5 ultramicrotome fitted with a diamond knife. The selected cell sheets were not additionally stained to avoid precipitates that could be confound with ETO NCs. Specimens were studied under a transmission electron microscope (JEM-100S, JEOL, Tokyo, Japan) at accelerating voltage of 80 kV.

### 10.2 Results

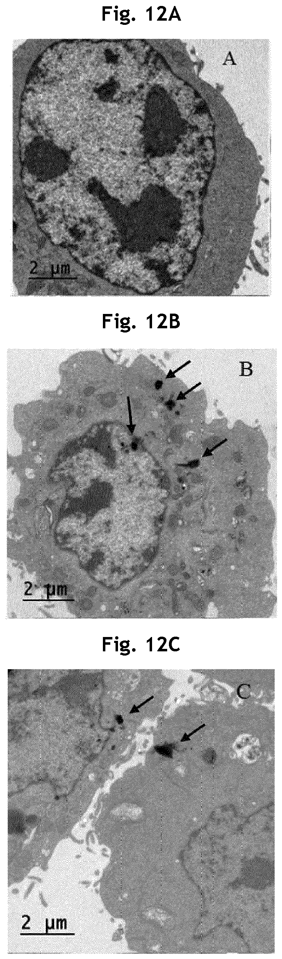

Cancer frequently acquires resistance to several drugs which is labelled as multidrug resistance (MDR) and represent a major drawback for cancer therapy, thus deliver a drug as nanoparticle to cancer cells could overcome MDR mechanisms. The mechanism of drug NC internalization has been evidenced to be among endocytosis pathways. *In vitro* cells imaging was performed with CT26 cells line to evidence whether ETO NC could be internalized into the cells as NC or solubilized drug; since the phagocyte mechanism is completely different according to the size, shape, charge surface and nature of the drug, it could have been caveolae or clathrin mediated endocytosis, pinocytosis or phagocytosis pathway. After two hours, TEM pictures displayed that NCs are internalized inside cells as lone particle in the cell perinuclear area. Also, despite the NC shape diversity did not influence the nanoparticle internalization as diverse shapes can be detected. Therefore, it may be concluded that ETO NC will be transported as single nanoparticles to the cancer cells in the blood stream at 37°C and not immediately solubilized after i.v. injection, hence changing the *in vivo* fate of NCs. Figure 12A shows control CT26 cells. Figure 12B and Figure 12C show the cell apoptosis beginning evidenced by the damaged membrane of the cells that were incubated with ETO NCs formulations.

### Example 11: Plasma and Tissues Pharmacokinetic

### 11.1 Material and Methods

Seventy-two BALB/c female (6 weeks) mice were used for the determination of the ETO NCs concentration overtime in the plasma and selected tissues (liver, rate, kidney, lungs). Four formulations were tested, ETO NCs/Pluronic F-127 0.2 % wt/v, ETO NCs/ F-127 0.2 % wt/v + albumin 0.48 % wt/v, Free ETO and Toposar^{®}. Each formulation was given intravenously at 10 mg/kg ETO.

Then, retro-orbital blood sample were realized (200 µL) at different time, 1, 15, 30, 45, 60 and 120 min and add in an Eppendorf tube containing 20 µL of EDTA. Plasma was collected by centrifugation at 2000 rpm for 15 min and frozen at -20°C for further high- performance liquid chromatography (HPLC) analysis. Tissues samples were taken after mice sacrifice at 45, 60 and 120 min in order to have enough drug accumulation in the selected organs and frozen at -80°C for further HPLC analysis. ETO contained in organs were recuperated by grinding organs in chloroform using Precellys^{®} tubes. Liver and kidneys were crushed in 5 mL of chloroform in a 7 mL capacity Precellys^{®} tubes, lungs and spleen in 1.2 mL of chloroform in a 2 mL capacity Precellys^{®} tubes. Samples were centrifuged and chloroform was totally evaporated in glass vials, dry residues were redispersed in 130 µL of water: methanol mixture as a mobile phase (50:50 v:v) and ready for analysis. The HPLC was set as reversed phase (RP-HPLC, 1260 Infinity, Agilent^{®}) with isocratic conditions. The analytical column was standard with a reversed phase C18 (250 mm × 4.6 mm, 5 µm, Waters). The injected volume was 50 µL for all samples.

### 11.2 Results

The ETO plasma concentration profile in mice was assessed and compared according to four different formulation of ETO. Two NCs formulation, the marketed product Toposar^{®} and the Free ETO. The pharmacokinetics results showed that both ETO NCs formulation experienced have significantly higher ETO plasma concentration than the Free ETO and the Toposar^{®} (p < 0.05) with an AUC₀₋₁₂₀ₘᵢₙ almost 2-fold greater and a higher mean residence time (p < 0.05). This is not the first time that NCs drug form were proved to have a better long-life time in C57BL/6 mice plasma than its solubilized analog. Ganta et al (Int. J. Pharm., vol. 367, no. 1-2, pp. 179-186, 2009) intravenously injected asulacrine NCs and also perceived a 2.7-fold lifespan augmentation in the plasma compared to the asulacrine solution. Besides the solid form of NCs increasing the plasma life time, the use of stabilizer comprising PEG is known to reduce protein binding and therefore extend the particle's plasma concentration. Regarding specifically ETO NCs/F-127 0.2% wt/v + Alb 0.48% wt/v, it was expected to have a significant better blood stream lifespan in comparison with ETO NCs/F-127 0.2% wt/v. Nanoparticles coated with albumin are well known to have an prolonged lifespan in blood as albumin has the ability to bind to the FcRn receptor protecting it from degradation, more specifically from endothelial catabolism. But in our study, ETO NCs with albumin had equivalent AUC₀₋₁₂₀ₘᵢₙ (550 ± 37.33 µg.min/mL) and MRT (2.75 ± 0.19 min) than ETO NCs (p > 0.05) stabilized with simply F-127.

**Table 6: Pharmacokinetics parameters of four etoposide formulation, average maximum mass in total mice plasma, area under the curve, half time (t_{1/2}) and mean residence time (MRT) based on Cₜ₌₀=200µg.**

| Formulations | Maximum amount in total mice plasma (**µg/mL**) ± SD (n=6) | AUC₀₋₁₂₀ₘᵢₙ (**µg.min/mL**) ± SD (n=6) | t_{1/2} (min) | MRT(min) ± SD (n=6) |
|---|---|---|---|---|
| Etoposide NCs/ F-127 0.2% wt/v | 58.22 ± 1.92 | 608 ± 66.84*-** | 5.52 | 3.04 ± 0.33 |
| Etoposide NCs/ F-127 0.2% wt/v + Albumin 0.48% wt/v | 48.22 ± 0.98 | 550 ± 37.33*'-**' | 5.70 | 2.75 ± 0.19 |
| Etoposide Solubilized | 37.62 ± 0.85 | 378 ± 29.24 | 4.89 | 1.89 ± 0.15 |
| Toposar^{®} | 37.22 ± 1.05 | 436 ± 42.59 | 5.52 | 2.18 ± 0.21 |

| | | | | |
|---|---|---|---|---|
| * p_{ETO NC-Toposar} < 0.05, ** P_{ETO NC-Free ETO}< 0.05; *', P_{ETO NC_Toposar} < 0.05, **' P_{ETO NC-Free ETO} < 0,05. Analysis was fit to one phase decay model. Statistical analysis was performed by two-way Anova with Bonferroni correction. | | | | |

### Example 12: Anticancer efficacy and hematological toxicity

### 12.1 Materials and methods

Sixty BALB/c female mice (Janvier, St Genest de Lisle, France) aged of 6 weeks were divided into 5 groups (5*n_{mice}=12), four groups received four different ETO formulations, ETO NCs /F-127 0.2% wt/v, ETO NCs /F-127 0.2% wt/v + albumin 0.48% wt/v, Free ETO, Toposar^{®} and one group were used as control. The first 4 groups have received 5 injections of ETO (4 different formulations) at 10 mg/kg, an injection daily for two consecutive days, a day for rest, followed by an injection daily for two days in a row. The untreated control group was used as comparison for tumor volume. Murine carcinoma tumors CT26 were subcutaneous confined on day 1 using a 12-gauge trocar (38 mm) into the mouse flank previously disinfected with alcohol. The anticancer treatment started on day 8 as described above to have homogeneous tumor growth in each group. Tumor size and body weight were evaluated using a digital caliper every two days until day 17. Tumors volume (V) were calculated as followed: V = (Length × Width *2)/2. For the survival study, weight loss superior to 10% or tumors size >10% of the mice body weight were established as endpoints. All mice were anesthetized before ETO injection, in an induction chamber under a flow of oxygen/isoflurane (30/70) (Tec 7, Minerve, Carnaxide, USA).

Thirty BALB/c female mice (Janvier, St Genest de Lisle, France) aged of 6 weeks were divided into 5 groups (5*n_{mice}=6), four groups received four different ETO formulations, ETO NCs /F-127 0.2% wt/v, ETO NCs /F-127 0.2% wt/v + albumin 0.48% wt/v, Free ETO, Toposar^{®} and one group were used as control. The drug schedule protocol was equivalent to the anticancer efficacy study. Blood samples were taken in the mice tail vein at 1, 12, 15, 17 and 22 days and transferred to Eppendorf tube containing 2 µL of EDTA for white blood cells (WBC) numbering using a MS9-5 (MS Pharmaceuticals, France). All mice were anesthetized before ETO injection, in an induction chamber under a flow of oxygen/isoflurane (30/70) (Tec 7, Minerve, Carnaxide, USA).

### 12.2 Results

The therapeutic efficacy following 4 different ETO treatment was evaluated on BALB/c female mice. ETO NCs/F-127 0.2% wt/v are significantly more profitable than the marketed product Toposar^{®} (p < 0.05). This is certainly justified by the size and the solid form of NCs that are EPR-shaped and have a longer blood stream lifespan. It has also been proven that nanoparticles have a better penetration into the surrounding interstitium of the tumor leading to a better bioavailability and thus anticancer efficacy. ETO NCs formulations were competed with different excipient composition to observe whether the effect on the tumor inhibition was major or not. At day 17, ETO NCs /F-127 0.2% wt/v was significantly better than ETO NCs /F-127 0.2% wt/v + albumin 0.48% wt/v (p < 0.05), indicating that low concentration of surfactant is more favorable for cancer treatment, and assuming that the nature of interactions and the force of the adsorptive bindings to the ETO NCs are dissimilar with the excipient composition and its concentration. For ETO NCs/F-127 0.2% wt/v, the time to reach the median tumor volume in comparison with Control was delayed of 5.31 days, slightly lower 3.27 days for ETO NCs/F-127 0.2% wt/v + albumin 0.48% wt/v respectively (Table 7). Toposar^{®} and Free ETO have barely postponed the median tumor volume of about half a day revealing a poor treatment response for these two formulations (Table 7). Regarding, the mice weight loss throughout the investigation, ETO NCs /F-127 0.2% wt/v had no weight loss proving that this treatment is well tolerated, nevertheless for the other two ETO NCs. For Toposar^{®} and Free ETO formulations a decrease is observed that can be primarily explained by a poor targeting of the ETO to the tumor and therefore to healthy organs. Also, a higher concentration of toxic excipients could foster unwanted side effect causing loss of appetite and thus the mice weight loss.

Blood samples were taken at day 1 as control, then at 12, 15, 17 and 22 days. The leukocyte count nadir were identified the last day of treatment for all groups (Table 8). ETO NC formulations and Toposar^{®} had a notable leukocyte decrease compared to the control ( p <0.05) the same day. The recovery of WBC from day 12 to day 22 was also equivalent for both ETO NC formulations in opposition with the Toposar^{®} (p > 0.05). No hematological significant change was observed for the NC formulations in comparison with Toposar^{®} (p < 0.05). In contrast, Liu et al injected solubilized ETO (World J. Gastroenterol, vol. 11, no. 31, pp. 4895-4898, 2005) at 5 mg/kg in Balb/c mice two days consecutively and observed a leucocyte decrease from 11 to 8 10⁹ /L (18% loss) that is in accordance with our protocol where ETO NCs were injected at 10 mg/kg four times and a leucocyte decrease from 11 to 2 10⁹ /L was observed (82% loss).

**Table 7: Antitumoral effect of etoposide nanocrystals, Toposar^{®}, Free etoposide**

| Treatment | Etoposide (mg/kg) - Schedule (day) | Mean body weight change (day of nadir) | Median Tumor Volume (mm³ on day 12) | %T/C^{a} | Time for median tumor to reach 50 mm³ | T-C (day)^{b} |
|---|---|---|---|---|---|---|
| Control | 10 mg/kg - d8, d9 and d11, d12 Etoposide (mg/kg) - Schedule (day) | - | 104 | - | 8.9 | - |
| ETO NCs/ F-127 0.2% wt/v | | -0.83 d9 | 30 | 71.2 | 14.21 | 5.31 |
| | | +4.79 d17 | | | | |
| ETO NCs/ F-127 0.2% + Alb 0.48% | | -2.49 d9 | 40 | 61.5 | 12.8 | 3.9 |
| | | +2.31 d17 | | | | |
| Free ETO | | -3.40 d10 | 69 | 33.7 | 9.5 | 0.6 |
| | | +3.85 d17 | | | | |
| Toposar^{®} | | -2.86 d13 | 105 | -1 | 9.5 | 0.6 |
| | | +0.41 d17 | | | | |

| | | | | | | |
|---|---|---|---|---|---|---|
| ^{a} Tumor growth inhibition ratio (% T/C) = ((MTVcontrol - MTVtreated groups) * MTVcontrol) *100 ; ^{b} Tumor growth inhibition delay in comparison to control (T-C) | | | | | | |

**Table 8 - White Blood Cells (WBCs) data recovered from adult female BALB/c Mice (n = 5) after an intravenous injection of four different etoposide formulation at 10 mg/kg. WBCs normality range 4-15 10⁹/L.**

| | **White blood cell count (10⁹ cells/L)** | | | | |
|---|---|---|---|---|---|
| Formulations | Days 1 | Days 12 | Days 15 | Days 17 | Days 22 |
| Control | 11.1 ± 2.1 | 6.0 ± 2.2 | 8.7 ± 2.1 | 10.6 ± 2.3 | 10.2 ± 3.1 |
| Etoposide NCs/ F-127 0.2% wt/v | 10.2 ±0.9 | 2.0 ± 1.8 | 5.7 ± 3.7 | 10.8 ± 2.3 | 10.0 ± 3.6 |
| Etoposide NCs/ F-127 0.2% wt/v + Albumin 0.48% wt/v | 11.1 ± 3.2 | 2.8 ± 1.0 | 8.2 ± 4.3 | 9.4 ± 3.3 | 8.3 ± 1.5 |
| Etoposide Solubilized | 8.9 ± 2.3 | 6.5 ± 4.4 | 11.6 ± 0.9 | 10.4 ± 1.3 | 11.3 ± 3.4 |
| Toposar^{®} | 8.7 ± 1.9 | 2.3 ± 1.1 | 8.9 ± 0.6 | 6.4 ± 0.8 | 9.8 ± 0.5 |

### Example 13: Preparation of podophyllotoxin nanocrystals

### 13.1 Material and methods

Podophyllotoxin as an API was dissolved in absolute methanol in glass vial and slowly injected under agitation (1200 rpm) in water containing no stabilizer P1.

The mixture was then precipitated by evaporating the entire solution using a rotovapor under vacuum for 0.5 h. The resulting powder was kept under vacuum to remove any traces of methanol, then hydrated with aqueous solution containing 5 mg of P407 as polymer P2 under 10 min sonication using a water-bath sonicator to engineer the nanocrystals dispersion. The nanosupension was then observed using TEM.

### 13.2 Results

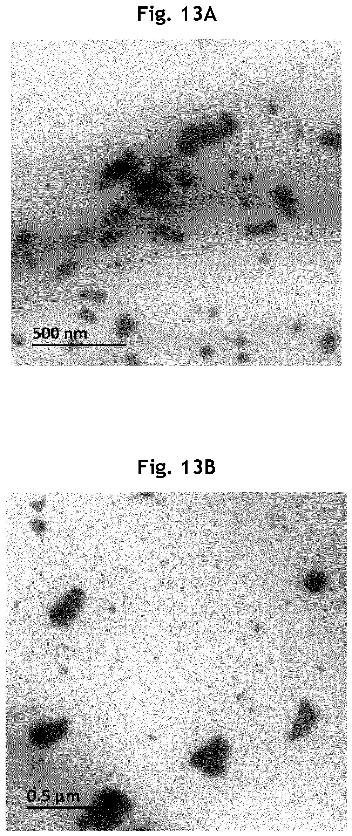

As may be seen of Figure 13A and Figure 13B, podophyllotoxin nanocrystals having an average size less than 500 nm are obtained.

## Claims

1. A method for manufacturing a nanostructured powder comprising nanocrystalline agglomerates containing active pharmaceutical ingredient (API) in its crystalline form, said method comprising:
(i) Preparing a first solution comprising API and an API solvent;
(ii) Mixing the first solution with a second solution comprising an API antisolvent and optionally a stabilizing agent P1 to obtain a miscible liquid mixture containing the API solvent, the API antisolvent, and solubilized API;
(iii) Evaporating said miscible liquid mixture until both the API solvent and the API antisolvent are evaporated, advantageously under vacuum;
**characterized in that**
- when the stabilizing agent P1 is present, the miscible liquid mixture has a stabilizing agent P1 to API weight ratio equal or less than 5, preferably less than 2,
- the API antisolvent volume to API solvent volume ratio is at least of 4.5, preferably at least of 5,
- the method does not comprise any intermediate step between steps (ii) and (iii), in particular any filtration or any other step intended to eliminate micrometric particles, and
- the API nanocrystals form and/or develop during step (iii).

2. The method according to claim 1 wherein the API is selected among podophyllotoxin, etoposide, teniposide, albendazole, amoitone B, amphotericin B, aprepitant, aripiprazole, ascorbyl palmitate, asulacrine, avanafil, azithromycin, baicalin, bexarotene, breviscapine, budenoside, buparvaquone, cabazitaxel, camptothecin, candesartan cilexetil, celecoxib, cilostazol, clofazimine, curcumin, cyclosporine, danazol, darunavir, dexamethasone, diclofenac acid, docetaxel, doxorubicin, efavirenz, fenofibrate, glibenclamide, griseofulvin, hesperetin, hydrocamptothecin, hydrocortisone, ibuprofen, indometacin, itraconazole, ketoprofen, loviride, lutein, mebendazole, mefenamic acid, meloxicam, methyltryptophan, miconazole, monosodium urate, naproxen, nimodipine, nisoldipine, omeprazole, oridonin, paclitaxel, piposulfan, prednisolone, puerarin, fisetin, resveratrol, riccardin D, rutin, silybin, simvastin, spironolactone, tarazepide and ziprasidone and mixtures thereof, preferably is selected among podophyllotoxin and podophyllotoxin derivatives such as etoposide and teniposide.

3. The method according to any one of claims 1 and 2 wherein the API solvent is selected among methanol, isopropanol, ethanol, acetonitrile, acetone, diethanolamine, diethylenetriamine, dimethylformamide, ethylamine, ethylene glycol, formic acid, furfuryl alcohol, glycerol, methyl diethanolamine, methyl isocyanide, N-Methyl-2-pyrrolidone, propanol, propylene glycol, pyridine, tetrahydrofuran, triethylene glycol and dimethyl sulfoxide, preferably selected from methanol, ethanol and acetonitrile, and more preferably methanol.

4. The method according to any one of claims 1 to 3 wherein the API antisolvent is selected among water, SH Buffer, HBS Buffer, PBS Buffer, an aqueous solution comprising glucose, sucrose, lactose, trehalose, NaCl, KCl, Na₂HPO₄, and/or KH₂PO₄, and mixtures thereof.

5. A nanostructured powder comprising nanocrystalline agglomerates containing API in its crystalline form, said agglomerates having at least one dimension of less than 1 µm.

6. The nanostructured powder according to claim 5 wherein the API is selected among podophyllotoxin and podophyllotoxin derivatives such as etoposide and teniposide.

7. The nanostructured powder according to any of claims 5 to 6 further comprising a stabilizing agent in a stabilizing agent to API weight ratio equal or less than 5, preferably less than 2.

8. The nanostructured powder according to any one of claims 5 to 6 wherein said powder is free of stabilizing agent.

9. A method for manufacturing a nanosuspension comprising API nanocrystals comprising a step of mixing a nanostructured powder as defined in any of claims 5 to 8, an API antisolvent and optionally a stabilizing agent P2 in a stabilizing agent P2 to API weight ratio equal or less than 5, preferably less than 2, wherein the nanostructured powder is optionally obtained from the method according to anyone of claims 1 to 5.

10. A nanosuspension obtainable by the method of claim 9 comprising API nanocrystals and an API antisolvent, wherein the API nanocrystals have an average size of less than 500 nm, preferably less than 200 nm, more preferably less than 100 nm.

11. The nanosuspension according to claim 10 wherein the weight ratio of stabilizing agent to API is equal or inferior to 5, preferentially comprised between 0 and 2.

12. The nanosuspension according to any of claims 10 to 11 wherein said API is selected among podophyllotoxin and podophyllotoxin derivatives such as etoposide and teniposide.

13. The nanosuspension according to any one of claims 10 to 12 wherein the stabilizing agent is selected among polysorbate 80, polyvinylpyrrolidone, hydroxypropylmethylcellulose, sodium lauryl sulfate, mannitol, lecithin, tocopheryl polyethylene glycol 1000 succinate, sorbitan trioleate 85, tyloxapol, poloxamer 338, sodium cholate, cholic acid, leucine, distearoyl-sn-glycero-3-phosphoethanolamine-N-[amino(polyethylene glycol)-1000, distearoyl-sn-glycero-3-phosphoethanolamine-N-[amino(polyethylene glycol)-2000, distearoyl-sn-glycero-3-phosphoethanolamine-N-[amino(polyethylene glycol)-5000, vitamin E, tragacanth, decyl glucoside, fetal calf serum, soy phosphatidylcholine, egg phosphatidylcholine, a synthetic derivative of phophatidylcholine, dioctyl sulfosuccinate, chitosan, maltose, Asialofetuin, transferrin, albumin, cyclodextrine, poloxamere 188, and poloxamere 407, preferably poloxamere 407.

14. A nanosuspension according to any of claims 10 to 13 for its use as a medicament, advantageously for treating cancer, more advantageously for treating a cancer selected from testicular cancer, lung cancer, lymphoma, leukemia, neuroblastoma, and ovarian cancer.

## Patentansprüche

1. Verfahren zum Herstellen eines nanostrukturierten Pulvers, das nanokristalline Agglomerate umfasst, die einen pharmazeutischen Wirkstoff (API) in seiner kristallinen Form enthalten, wobei das Verfahren Folgendes umfasst:
(i) Vorbereiten einer ersten Lösung, die API und ein API-Lösungsmittel umfasst;
(ii) Mischen der ersten Lösung mit einer zweiten Lösung, die ein API-Antilösungsmittel und optional ein Stabilisierungsmittel P1 umfasst, um eine mischbare Flüssigkeitsmischung zu erhalten, die das API-Lösungsmittel, das API-Antilösungsmittel und löslich gemachten API enthält;
(iii) Verdampfen der mischbaren Flüssigkeitsmischung, bis sowohl das API-Lösungsmittel als auch das API-Antilösungsmittel verdampft sind, vorteilhafterweise unter Vakuum;
**dadurch gekennzeichnet, dass**
- wenn das Stabilisierungsmittel P1 vorhanden ist, die mischbare Flüssigkeitsmischung ein Gewichtsverhältnis von Stabilisierungsmittel P1 zu API von kleiner gleich 5, vorzugsweise kleiner als 2, aufweist,
- das Verhältnis von API-Antilösungsmittelvolumen zu API-Lösungsmittelvolumen mindestens 4,5, vorzugsweise mindestens 5, beträgt,
- das Verfahren keinen Zwischenschritt zwischen den Schritten (ii) und (iii), insbesondere keine Filtration oder keinen anderen Schritt, der zur Entfernung mikrometrischer Partikel vorgesehen ist, umfasst, und
- die API-Nanokristalle sich während Schritt (iii) bilden und/oder entwickeln.

2. Verfahren nach Anspruch 1, wobei der API ausgewählt wird aus Podophyllotoxin, Etoposid, Teniposid, Albendazol, Amoiton B, Amphotericin B, Aprepitant, Aripiprazol, Ascorbylpalmitat, Asulacrin, Avanafil, Azithromycin, Baicalin, Bexaroten, Breviscapin, Budenosid, Buparvaquon, Cabazitaxel, Camptothecin, Candesartan Cilexetil, Celecoxib, Cilostazol, Clofazimin, Curcumin, Cyclosporin, Danazol, Darunavir, Dexamethason, Diclofenacsäure, Docetaxel, Doxorubicin, Efavirenz, Fenofibrat, Glibenclamid, Griseofulvin, Hesperetin, Hydrocamptothecin, Hydrocortison, Ibuprofen, Indometacin, Itraconazol, Ketoprofen, Lovirid, Lutein, Mebendazol, Mefenaminsäure, Meloxicam, Methyltryptophan, Miconazol, Mononatriumurat, Naproxen, Nimodipin, Nisoldipin, Omeprazol, Oridonin, Paditaxel, Piposulfan, Prednisolon, Puerarin, Fisetin, Resveratrol, Riccardin D, Rutin, Silybin, Simvastin, Spironolacton, Tarazepid und Ziprasidon und Mischungen davon, vorzugsweise ausgewählt aus Podophyllotoxin und Podophyllotoxin-Derivaten wie Etoposid und Teniposid.

3. Verfahren nach einem der Ansprüche 1 und 2, wobei das API-Lösungsmittel ausgewählt wird aus Methanol, Isopropanol, Ethanol, Acetonitril, Aceton, Diethanolamin, Diethylentriamin, Dimethylformamid, Ethylamin, Ethylenglykol, Ameisensäure, Furfurylalkohol, Glycerin, Methyldiethanolamin, Methylisocyanid, N-Methyl-2-pyrrolidon, Propanol, Propylenglykol, Pyridin, Tetrahydrofuran, Triethylenglykol und Dimethylsulfoxid, vorzugsweise ausgewählt aus Methanol, Ethanol und Acetonitril und besonders bevorzugt Methanol.

4. Verfahren nach einem der Ansprüche 1 bis 3, wobei das API-Antilösungsmittel ausgewählt wird aus Wasser, SH-Puffer, HBS-Puffer, PBS-Puffer, einer wässrigen Lösung, die Glucose, Saccharose, Lactose, Trehalose, NaCl, KCl, Na₂HPO₄ und/oder KH₂PO₄ umfasst, und Mischungen davon.

5. Nanostrukturiertes Pulver, das nanokristalline Agglomerate umfasst, die ein API in seiner kristallinen Form enthalten, wobei die Agglomerate mindestens eine Größe von weniger als 1 µm aufweisen.

6. Nanostrukturiertes Pulver nach Anspruch 5, wobei der API ausgewählt wird aus Podophyllotoxin und Podophyllotoxin-Derivaten wie Etoposid und Teniposid.

7. Nanostrukturiertes Pulver nach einem der Ansprüche 5 bis 6, das ferner ein Stabilisierungsmittel in einem Gewichtsverhältnis von Stabilisierungsmittel zu API von kleiner gleich 5, vorzugsweise weniger als 2, umfasst.

8. Nanostrukturiertes Pulver nach einem der Ansprüche 5 bis 6, wobei das Pulver frei von Stabilisierungsmitteln ist.

9. Verfahren zum Herstellen einer API-Nanokristalle umfassenden Nanosuspension, das einen Schritt des Mischens eines nanostrukturierten Pulvers gemäß einem der Ansprüche 5 bis 8, eines API-Antilösungsmittels und optional eines Stabilisierungsmittels P2 in einem Gewichtsverhältnis von Stabilisierungsmittel P2 zu API von kleiner oder gleich 5, vorzugsweise kleiner als 2, umfasst, wobei das nanostrukturierte Pulver optional aus dem Verfahren nach einem der Ansprüche 1 bis 5 erhalten wird.

10. Nanosuspension, die durch das Verfahren nach Anspruch 9 erhalten werden kann, umfassend API-Nanokristalle und ein API-Antilösungsmittel, wobei die API-Nanokristalle eine durchschnittliche Größe von weniger als 500 nm, vorzugsweise weniger als 200 nm, besonders bevorzugt weniger als 100 nm aufweisen.

11. Nanosuspension nach Anspruch 10, wobei das Gewichtsverhältnis von Stabilisierungsmittel zu API kleiner oder gleich 5 ist und vorzugsweise zwischen 0 und 2 beträgt.

12. Nanosuspension nach einem der Ansprüche 10 bis 11, wobei der API ausgewählt wird aus Podophyllotoxin und Podophyllotoxin-Derivaten wie Etoposid und Teniposid.

13. Nanosuspension nach einem der Ansprüche 10 bis 12, wobei das Stabilisierungsmittel ausgewählt wird aus Polysorbat 80, Polyvinylpyrrolidon, Hydroxypropylmethylcellulose, Natriumlaurylsulfat, Mannitol, Lecithin, Tocopheryl-Polyethylenglykol-1000-Succinat, Sorbitantrioleat 85, Tyloxapol, Poloxamer 338, Natriumcholat, Cholsäure, Leucin, Distearoyl-sn-glycero-3-phosphoethanolamin-N-[amino(polyethylenglykol)-1000, Distearoyl-sn-glycero-3-phosphoethanolamin-N-[amino(polyethylenglykol)-2000, Distearoyl-sn-glycero-3-phosphoethanolamin-N-[amino(polyethylenglykol)-5000, Vitamin E, Traganth, Decylglucosid, fötalem Kälberserum, Soja-Phosphatidylcholin, Ei-Phosphatidylcholin, einem synthetischen Derivat von Phosphatidylcholin, Dioctylsulfosuccinat, Chitosan, Maltose, Asialofetuin, Transferrin, Albumin, Cydodextrin, Poloxamer 188 und Poloxamer 407, vorzugsweise Poloxamer 407.

14. Nanosuspension nach einem der Ansprüche 10 bis 13 zur Verwendung als Medikament, vorteilhafterweise zur Behandlung von Krebs, noch vorteilhafter zur Behandlung eines Krebses, der aus Hodenkrebs, Lungenkrebs, Lymphom, Leukämie, Neuroblastom und Eierstockkrebs.

## Revendications

1. Procédé de fabrication d'une poudre nanostructurée comprenant des agglomérats nanocristallins contenant un principe pharmaceutique actif (API) sous sa forme cristalline, ledit procédé comprenant :
(i) la préparation d'une première solution comprenant l'API et un solvant d'API ;
(ii) le mélange de la première solution avec une deuxième solution comprenant un anti-solvant d'API et facultativement un agent stabilisant P1 pour obtenir un mélange liquide miscible contenant le solvant d'API, l'anti-solvant d'API, et l'API solubilisé ;
(iii) l'évaporation dudit mélange liquide miscible jusqu'à ce que le solvant d'API et l'anti-solvant d'API soient évaporés, de manière avantageuse sous vide ;
**caractérisé en ce que**
- lorsque l'agent stabilisant P1 est présent, le mélange liquide miscible présente un rapport en poids entre agent stabilisant P1 et API égal ou inférieur à 5, de préférence inférieur à 2,
- le rapport entre volume d'anti-solvant d'API et volume de solvant d'API est d'au moins 4,5, de préférence d'au moins 5,
- le procédé ne comprend aucune étape intermédiaire entre les étapes (ii) et (iii), en particulier aucune filtration ou autre étape quelconque destinée à éliminer des particules micrométriques, et
- les nanocristaux d'API se forment et/ou se développent lors de l'étape (iii).

2. Procédé selon la revendication 1 dans lequel l'API est choisi parmi la podophyllotoxine, étoposide, téniposide, albendazole, amoitone B, amphotéricine B, aprépitant, aripiprazole, palmitate d'ascorbyle, asulacrine, avanafil, azithromycine, baicaline, bexarotène, bréviscapine, budénoside, buparvaquone, cabazitaxel, camptothécine, candésartan cilexétil, célécoxib, cilostazol, clofazimine, curcumine, cyclosporine, danazol, darunavir, dexaméthasone, acide diclofénac, docétaxel, doxorubicine, éfavirenz, fénofibrate, glibenclamide, griséofulvine, hespérétine, hydrocamptothécine, hydrocortisone, ibuprofène, indométacine, itraconazole, kétoprofène, loviride, lutéine, mébendazole, acide méfénamique, méloxicam, méthyltryptophane, miconazole, urate monosodique, naproxène, nimodipine, nisoldipine, oméprazole, oridonine, paclitaxel, piposulfan, prednisolone, puérarine, fisétine, resvératrol, riccardine D, rutine, silybine, simvastine, spironolactone, tarazépide et ziprasidone et des mélanges de ceux-ci, est de préférence choisi parmi la podophyllotoxine et des dérivés de podophyllotoxine tels que l'étoposide et le téniposide.

3. Procédé selon l'une quelconque des revendications 1 et 2 dans lequel le solvant d'API est choisi parmi du méthanol, isopropanol, éthanol, acétonitrile, acétone, diéthanolamine, diéthylènetriamine, diméthylformamide, éthylamine, éthylène glycol, acide formique, alcool furfurylique, glycérol, méthyldiéthanolamine, isocyanure de méthyle, N-méthyl-2-pyrrolidone, propanol, propylène glycol, pyridine, tétrahydrofurane, triéthylène glycol et diméthylsulfoxyde, de préférence choisi parmi du méthanol, éthanol et acétonitrile, et plus préférablement du méthanol.

4. Procédé selon l'une quelconque des revendications 1 à 3 dans lequel l'anti-solvant d'API est choisi parmi l'eau, Tampon SH, Tampon HBS, Tampon PBS, une solution aqueuse comprenant du glucose, saccharose, lactose, tréhalose, NaCl, KCl, Na₂HPO₄, et/ou KH₂PO₄, et des mélanges de ceux-ci.

5. Poudre nanostructurée comprenant des agglomérats nanocristallins contenant un API sous sa forme cristalline, lesdits agglomérats ayant au moins une dimension inférieure à 1 µm.

6. Poudre nanostructurée selon la revendication 5 dans laquelle l'API est choisi parmi la podophyllotoxine et des dérivés de podophyllotoxine tels que l'étoposide et le téniposide.

7. Poudre nanostructurée selon l'une quelconque des revendications 5 à 6 comprenant en outre un agent stabilisant selon un rapport en poids entre agent stabilisant et API égal ou inférieur à 5, de préférence inférieur à 2.

8. Poudre nanostructurée selon l'une quelconque des revendications 5 à 6, dans laquelle ladite poudre est exempte d'agent stabilisant.

9. Procédé de fabrication d'une nanosuspension comprenant des nanocristaux d'API comprenant une étape de mélange d'une poudre nanostructurée telle que définie selon l'une quelconque des revendications 5 à 8, d'un anti-solvant d'API et facultativement d'un agent stabilisant P2 selon un rapport en poids entre agent stabilisant P2 et API égal ou inférieur à 5, de préférence inférieur à 2, dans lequel la poudre nanostructurée est facultativement obtenue à partir du procédé selon l'une quelconque des revendications 1 à 5.

10. Nanosuspension pouvant être obtenue par le procédé selon la revendication 9 comprenant des nanocristaux d'API et un anti-solvant d'API, dans laquelle les nanocristaux d'API ont une taille moyenne inférieure à 500 nm, de préférence inférieure à 200 nm, plus préférablement inférieure à 100 nm.

11. Nanosuspension selon la revendication 10 dans laquelle le rapport en poids entre agent stabilisant et API est égal ou inférieur à 5, de préférence compris entre 0 et 2.

12. Nanosuspension selon l'une quelconque des revendications 10 à 11, dans laquelle ledit API est choisi parmi la podophyllotoxine et des dérivés de podophyllotoxine tels que l'étoposide et le téniposide.

13. Nanosuspension selon l'une quelconque des revendications 10 à 12 dans laquelle l'agent stabilisant est choisi parmi du polysorbate 80, polyvinylpyrrolidone, hydroxypropylméthylcellulose, laurylsulfate de sodium, mannitol, lécithine, succinate de tocophéryle polyéthylène glycol 1000, trioléate de sorbitan 85, tyloxapol, poloxamer 338, cholate de sodium, acide cholique, leucine, distéaroyl-sn-glycéro-3-phosphoéthanolamine-N-[amino(polyéthylène glycol)-1000, distéaroyl-sn-glycéro-3-phosphoéthanolamine-N-[amino(polyéthylèneglycol)-2000, distearoyl-sn-glycéro-3-phosphoéthanolamine-N-[amino(polyéthylèneglycol)-5000, vitamine E, adragante, décyle glucoside, sérum de veau foetal, phosphatidylcholine de soja, phosphatidylcholine d'œuf, un dérivé synthétique de phophatidylcholine, sulfosuccinate de dioctyle, chitosane, maltose, asialofetuine, transferrine, albumine, cyclodextrine, poloxamer 188, et poloxamer 407, de préférence le poloxamer 407.

14. Nanosuspension selon l'une quelconque des revendications 10 à 13 pour son utilisation comme médicament, de manière avantageuse pour traiter un cancer, de manière plus avantageuse pour traiter un cancer choisi parmi un cancer des testicules, cancer du poumon, lymphome, leucémie, neuroblastome, et cancer de l'ovaire.
